(19) Europäisches Patentamt
European Patent Office
Office européen des brevets

(11) EP 1 452 605 A1

(12) EUROPEAN PATENT APPLICATION

(43) Date of publication:
**01.09.2004 Bulletin 2004/36**

(51) Int Cl.[7]: **C12Q 1/25**

(21) Application number: **03075596.1**

(22) Date of filing: **27.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicants:
- **Universiteit Utrecht Holding B.V.**
**3584 CL Utrecht (NL)**
- **UMC Utrecht Holding B.V.**
**3584 CK Utrecht (NL)**

(72) Inventor: **Nottet, Johannes S. L. M.**
**1095 DX Amsterdam (NL)**

(74) Representative:
**Prins, Adrianus Willem, Mr. Ir. et al**
**Vereenigde,**
**Nieuwe Parklaan 97**
**2587 BN Den Haag (NL)**

# (54) Method for determining antiviral activity

(57) The invention relates to the field of antiviral agents, and more specifically to antiviral therapy. The invention provides use of at least one compound or mixture of compounds of the general formula or a functional equivalent or pharmaceutically acceptable salt or hydrate thereof for its likelihood to modulate a viral polymerase, e.g. reverse transcriptase.

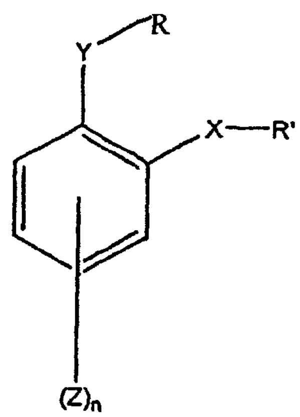

EP 1 452 605 A1

## Description

The invention relates to the field of antiviral agents, and more specifically to antiviral therapy.

One of the great paradoxes of medicine is that the simplest of organisms are the most difficult to control. While great progress has been made in controlling more complex organisms, for example bacteria, with hundreds of different antibacterial pharmaceutical compositions or antibiotics, there are very few pharmaceutical compositions intended or adapted for antiviral therapy that are of proven effectiveness. The major drawback in developing antiviral agents has been an inability to distinguish viral replicative mechanisms from host replicative processes. Nevertheless, progress has been made over the past two decades in discovering molecules necessary for virus replication, in characterising them mechanistically, and in developing antiviral agents to inhibit them (for review see Hirsch et al., In: Fields Virology, Chapter 15, Lippincot-Raven Publishers, 1996). Well known antiviral agents are for example amantadine and rimandatine and other anti-influenza agents, acyclovir, gangcyclovir and related agents, foscarnet and other anti-herpesvirus agents, ribavirine and various antiretroviral agents.

Progress and understanding in the field of antiretroviral therapy in the past 3 years has been dramatic (for review see Hammer and Yeni. AIDS, 12:S181-S188, 1998). Progress has been fuelled by three major advances. First, increasing knowledge of disease pathogenesis has provided underpinnings for current therapeutic rationale. The proliferative nature of the viral replicative process ($10^{10}$ virions produced and destroyed each day), the rapid viral turnover (virion plasma half-life of 6 h or less), and the recognition of second and third phases of viral decay under the influence of potent antiretroviral therapy resulting from the presence of longer-lived cell reservoirs has guided the current principles of antiretroviral therapy. The second advance has been the widening array of therapeutic choices represented by the increasing numbers of agents available to patients and clinicians. Third, the availability of increasingly sophisticated patient monitoring techniques, such as viral load determinations, has simultaneously provided the tools for dissecting HIV disease pathogenesis and monitoring the effects of treatment in affected individuals. Taken together, these developments have led to the generally accepted principle that potent combination regimens (also called highly active antiviral therapy or HAART) designed to drive and maintain plasma HIV-RNA concentration below the limits of detection of currently available assays are the treatments of choice.

However, a number of practical limitations to this idealised approach have increasingly been recognised. These include: the variability of initial virologic response according to the disease stage, particularly the high rate of failure in those with advanced HIV infection; the challenge of patient adherence to complex regimens; drug failure and the threat of multidrug resistance; the lack of predictably effective salvage therapies; the emergence of longer-term toxicities to the protease inhibitor class of compounds; and the sharpening division between populations of the world related to cost and access to effective agents.

In several countries there are 11 agents approved for the treatment of HIV infection and the reasonable expectation is that the total will rise to 15 shortly. These agents are either HIV reverse transcriptase inhibitors of the nucleoside, non-nucleoside, and nucleotide subclasses or members of the HIV-protease inhibitor class. Although the simple calculation of the number of two-, three- and four-drug combinations would suggest that the regimen choices for initial and alternative therapies are vast, in reality they are much more limited as a result of cross-resistance, toxicities, tolerance, drug or food interactions and other practical considerations. Thus, although it is true that the options for initial potent, combination regimens are increasing, when one considers the limitations on subsequent regimens conferred by the initial choice, one realises the restricted options for long-term virologic suppression that currently exist.

In areas where drug access is not a problem, the current recommended standard for initial therapy is a potent *in vivo* protease inhibitor combined with two nucleoside analogs with the first alternative being a non-nucleoside reverse transcriptease inhibitor in combination with two nucleoside analogs. However, the emergence of drug resistance during treatment and its association with treatment failure have been described with nearly all of the antiretroviral agents in use or in development. Therefore, resistance testing might be thought to logically assist with the choice of alternative treatment in the setting of treatment failure and assist with the choice of initial therapy when primary drug resistance is suspected. However, there are many questions that need to be answered before resistance testing (either genotypic or phenotypic) becomes accepted as a routine clinical tool. In what setting and to what extent this technology will improve decision making is not clear and drug resistance is only one of a number of reasons for treatment failure. Resistance testing results are most reflective of the selective pressure of the current drug that might emerge quickly on a new regimen. Further, one cannot always deduce the phenotypic susceptibility of a viral strain from its genotype because of assay sensitivity and resistance mutational interactions. Cross-resistance, particularly to protease inhibitors, may also be a dynamic process in which viruses are "primed" by mutations selected on a previous therapy to develop resistance more quickly when exposed to a new member of the same drug class.

Failure of a particular antiretroviral drug regimen may be defined clinically, immunologically or virologically. Increasingly, for individuals on their initial drug combination, a strict definition of failure is being applied, that is, detectable viremia following previous suppression below the detection below the detection limit of the assay being employed. With the advent of plasma HIV-RNA assays with detection limits at the approximate 50 copies/ml range, this has raised

the question of whether a confirmed rise above this threshold should trigger a treatment change given the still limited therapeutic armamentarium.

The advances and the limitations of the currently available antiretroviral agents make it clear that new agents and combinations are urgently needed. Now available are four new agents: abacavir(a nucleoside analog reverse transcriptase inhibitor), efavirenz (a non-nucleoside reverse transcriptase inhibitor), adefovir dipivoxil (a nucleotide reverse transcriptase inhibitor), and amprenavir (a protease inhibitor). These agents will carry with them an increasing number of choices for patients and clinicians but are most likely to benefit antiretroviral-naive or minimally drug-experienced individuals only. Their role in "salvage" regimens is currently under investigation but the potential for cross-resistance with the currently approved agents may well limit their effectiveness in this circumstance.

Earlier (WO01/01985 and WO01/68086) the inventors provided use of a *o*-(acetoxyphenyl)hept-2-ynyl sulfide (APHS) or of a APHS-like compound of the general formula

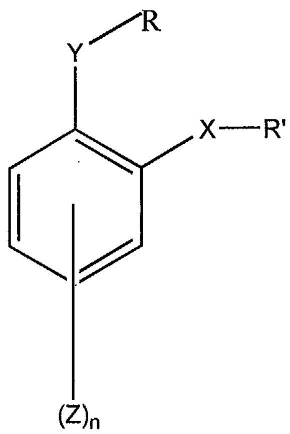

wherein X and Y are independently O, S, SO, $SO_2$, $SO_3$, but preferably O,S, $SO_2$; wherein n is 0, 1, 2, 3, 4, but preferably 1 or 2; wherein R, R' are independently H with the proviso that when R is H, R' is not H, a $C_1$-$C_{10}$, branched or unbranched, substituted or unsubstituted (preferably the substitute is one or more of halogen or $CF_3$), saturated or (poly) unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, $CH_3$, $CF_3$, $CH_2CL$, $CH_2Br$, $CH_2CH_3$, $(CH_2)_2CH_3$, $(CH_2)_3CH_3$, $(CH_2)_4CH_3$, $(CH_2)_5CH_3$, $(CH_2)_6CH_3$, $CH(CH_3)_2$, $CH(CH_3)_3$, $CH{=}C{=}CH_2$, $(CH_2)_2O(CH_2)_3CH_3$, $CH_2HC{=}CH(CH_2)_3CH_3$, $CH_2C{\equiv}C(CH_2)_3CH_3$, $CH_2C{\equiv}C(CH_2)_2CH_3$, $CH_2C{\equiv}C\text{-}CCH_2CH_3$, $CH_2C{\equiv}C\text{-}CH_3$ and $CH_2C{\equiv}CH$ and isomers or homologues thereof; and wherein R' is R, preferably selected from the group consisting of H, $CH_3$,; and wherein R or R' may contain ether linkages or carbonyl or thiocarbonyl functions attached to the ring structure such as ring-(C=O/S)-R/R'
and Z is independently R, R', XR, XR', YR or YR'
or a functional equivalent thereof for the production of a pharmaceutical composition for the treatment of a viral infection.

The present inventors also provided use of at least one APHS-like compound or mixture of compounds of the general formula

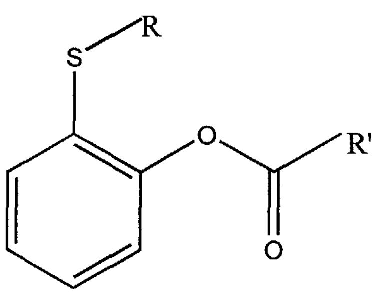

or a functional equivalent or pharmaceutically acceptable salt or hydrate thereof for the production of a pharmaceutical composition for the treatment of a viral infection. In WO01/01985 and WO01/68086 it was postulated that a compound of said general formula or a functional equivalent thereof antagonizes activities of transcription factors such as AP-1, STAT and NF-κB, assumedly with the effect that viral functions such as virus transcription and/or viral gene expression were functionally inhibited

The invention provides a method for determining anti-viral activity of a compound, in particular of AHPS or modifications or derivatives thereof, e.g. a compound of the general formula as indicated below or a derivative or modification thereof

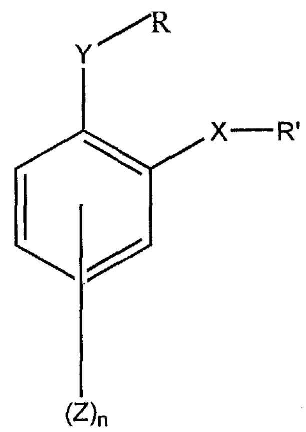

wherein X and Y are independently O, S, SO, $SO_2$, $SO_3$, but preferably O,S, $SO_2$; wherein n is 0, 1, 2, 3, 4, but preferably 1 or 2; wherein R, R' are independently H with the proviso that when R is H, R' is not H, a $C_1$-$C_{10}$, branched or unbranched, substituted or unsubstituted (preferably the substitute is one or more of halogen or $CF_3$), saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, $CH_3$, $CF_3$, $CH_2CL$, $CH_2Br$, $CH_2CH_3$, $(CH_2)_2CH_3$, $(CH_2)_3CH_3$, $(CH_2)_4CH_3$, $(CH_2)_5CH_3$, $(CH_2)_6CH_3$, $CH(CH_3)_2$, $CH(CH_3)_3$, $CH{=}C{=}CH_2$, $(CH_2)_2O(CH_2)_3CH_3$, $CH_2HC{=}CH(CH_2)_3CH_3$, $CH_2C{\equiv}C(CH_2)_3CH_3$, $CH_2C{\equiv}C(CH_2)_2CH_3$, $CH_2C{\equiv}C\text{-}CCH_2CH_3$, $CH_2C{\equiv}C\text{-}CH_3$ and $CH_2C{\equiv}CH$ and isomers or homologues thereof; and wherein in a particular embodiment R' is R, preferably selected from the group consisting of H, $CH_3$,; and wherein X and/or Y may be linked to R or R' via ether linkages or carbonyl or thiocarbonyl functions such as -(C=O/S)-, wherein R or R' may carry hydroxyl groups, and wherein Z is independently R, R', XR, XR', YR or YR' ,

or a functional equivalent or pharmaceutically acceptable salt or hydrate of said compound for its likelihood to modulate a viral polymerase. Replacements or substitutions of said general formula for example comprise omitting X and/or Y, replacing S with O, Se or Te, and/or additionally substituting the ring with one or more side groups such as R or R'. It is herein provided to test above compound or a modification derivative of an above provided compound for its likelihood to modulate a viral polymerase. Such a modified compound for example comprises a compound according to the general formula as indicated above wherein, however, X and/or Y are omitted and Rand/or R' are directly linked to the ring structure and/or wherein R and/or R' are linked to each other. We for example found that o-(acetoxyphenyl)hept-2-ynyl sulfide (APHS) and analogues, can inhibit HIV-1 replication. When administered during the first steps of the infection, APHS was capable of inhibiting the replication of several HIV-1 strains (macrophage-tropic and/or lymphocytotropic) in a dose-dependent manner in both peripheral blood mononuclear cells (PBMC), monocyte-derived macrophages and peripheral blood lymphocytes with 50% inhibitory concentration values of approximately 10 microM. It was found that APHS did not affect HIV-1 replication once the provirus was already inserted into the cellular genome. APHS did also not inhibit HIV-1 entry into host cells as determined by quantification of gag RNA inside PBMC 2h after infection. However, APHS did inhibit gag DNA synthesis during reverse transcription in primary cells, which indicates that APHS targets the a polymearse, e.g. the reverse transcription process. Reverse transcriptase is a key target for antiviral AIDS therapy. It performs a crucial step in viral reproduction -- replicating the single-stranded viral RNA genome to double-stranded DNA. Known inhibitors of reverse transcriptase fall into two categories: chain terminators (e.g. AZT), and non-nucleoside site binding agents(e.g. nevirapine). Unfortunately, viral resistance develops for both these classes in as little as 6 weeks, which compromises their effectiveness as drugs. Thus there is a need to develop new classes of inhibitors to reverse transcriptase. The invention provides a method to rationally design compounds effective as anti-viral agent directed against polymerases such as HIV-1 reverse transcriptase (RT). RT is a key enzyme in the HIV-1 life cycle, converting single stranded viral RNA into double stranded DNA. RT contains two active sites: a polymerase site, which extends a DNA primer from either RNA or DNA template; and a ribonuclease H (RNaseH) site, which

degrades RNA from an RNA-DNA duplex. The polymerase domain is described by an analog to a hand, with palm, thumb, fingers, and connection subdomains. The crystal structure contains a DNA binding groove in the heterodimer, in which the DNA is "grasped" at one end by the hand and continues to the RNaseH domain. (see for example http://www.cmpharm.ucsf.edu/kuntz/). In a preferred embodiment provides the invention provides a method to rationally design compounds effective against a polymerase comprising use of a compound of the general formula as above or a pharmaceutically acceptable salt or hydrate thereof wherein R is H, $CF_3$ or a C1-C10 (but due to its increased lipophilicity preferably a C4-C10, branched or unbranched, substituted or unsubstituted (preferably the substitute is a halogen), saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, $CH_3$, $CH_2CH_3$, $(CH_2)_2CH_3$, $(CH_2)_3CH_3$, $(CH_2)_4CH_3$, $(CH_2)_5CH_3$, $(CH_2)_6CH_3$, $CH(CH_3)_2$, $CH(CH_3)_3$, $CH{=}C{=}CH_2$, $(CH_2)_2O(CH_2)_3CH_3$, $CH_2HC{=}CH(CH_2)_3CH_3$, $CH_2C{\equiv}C(CH_2)_3CH_3$, $CH_2C{\equiv}C(CH_2)_2CH_3$, $CH_2C{\equiv}C\text{-}CCH_2CH_3$, $CH_2C{\equiv}C\text{-}CH_3$ AND $CH_2C{\equiv}CH$ and isomers or homologues thereof; and wherein R' is R, preferably selected from the group consisting of H, $CH_3$, $CF_3$, $CH_2CL$ and $CH_2Br$. *o*-(acetoxyphenyl)hept-2-ynyl sulfide (APHS) inhibits HIV-1 replication by interfering with the reverse transcription process. Therewith, the invention provides APHS modifications or derivatives that are even more potent against viral infections such as those with HIV-1. For example, substitution of the acetate group at position 1' by an hydroxyl group and substitution of the rigid 2-heptynylthio group at position 2' by the more flexible pentylthio group originated a compound with an $IC_{50}$ of 0.9 microM, a $TC_{50}$ of 149 microM and an SI of 159.

The present invention also provides a pharmaceutical composition intended and adapted for the treatment of a viral infection (herein also called an anti-viral agent) comprising at least one compound or a mixture of compounds according to said general formula (preferably selected with a method as provided herein) and a pharmaceutically acceptable carrier of diluent, found active against a polymerase as provided herein. In order to use a compound according to said general formula or a pharmaceutically acceptable salt or hydrate thereof in therapy, it will normally be formulated into a pharmaceutical composition in accordance with standard pharmaceutical practice. This invention, therefore, also relates to a pharmaceutical composition for the treatment of a viral infection comprising an effective amount of a compound according to said general formula having anti-polymerase activity, or a pharmaceutically acceptable salt or hydrate thereof, and pharmaceutically acceptable carrier or diluent. A pharmaceutical composition having anti-polymerase activity as provided by the invention allows treatment in a conveniently wide therapeutic window

In a preferred embodiment, the invention provides a use according to the invention wherein said viral infection is caused by a virus at least partly resistant against treatment with another antiviral agent such as for example shown in the detailed description.

In a preferred embodiment, the invention provides use according to the invention wherein said viral infection comprises a retroviral infection. Such a retroviral infection can for example comprise a leukemia virus infection, such as caused by bovine leukemia virus or human T-cell-leukemia virus. Other retroviral infections known in the art are for example ovine lentivirus infections or spumaretrovirus infections. Also, such a retroviral infection can comprise an infection with a recombinant retrovirus which is for example constructed for use in gene therapy. Preferably, the invention provides use according to the invention wherein said retroviral infection is caused by a immunodeficiency virus such as human or simian immunodeficiency virus (HIV or SIV). Current anti-HIV compounds are directed against various stages in the HIV-1 life cycle. For instance, the nucleoside and non-nucleoside analogs are directed against reverse transcriptase, the enzyme that converts the viral RNA into DNA. In such a way virions released by HIV-infected cells are not infectious for other target cells, unless mutations in the reverse transcriptase occur that confer resistance to these classes of anti-HIV drugs. Another class of anti-HIV compounds that is part of all new triple anti-HIV therapy treatment regimens are the protease inhibitors. These compounds likely prevent the formation of complete virions by HIV-infected cells and thus are intended to prevent the spread of HIV-1 to new target cells.

The invention furthermore provides use according to the invention wherein said treatment additionally comprises treatment with another pharmaceutical composition, preferably selected for its synergistic action with a compound as provided herein. For example, combinatorial therapy to treat virus infections, as is often the case when HIV-infected individuals are treated is now provided wherein said other pharmaceutical composition at least comprises an antiviral agent, such as for example amantadine and rimandatine or another anti-influenza agents, acyclovir, gangcyclovir or related agent, foscarnet or other anti-herpesvirus agent, ribavirine or a antiretroviral agent, or an antiviral agent as provided by the invention. Of course, combination therapies including an anti-viral agent according to the invention, and additionally comprising more than one additional anti-viral agent, such as combinations of said anti-viral agent as provided by the invention with nucleoside analogue reverse transcriptase inhibitors and/or with non-nucleoside reverse transcriptase inhibitors and/or with nucleotide analogue reverse transcriptase inhibitors and/or with protease inhibitors is provided as well, also since an anti-viral agent as provided by the invention is particularly effective against otherwise drug-resistant viruses.

Furthermore, the invention provides a pharmaceutical composition intended and adapted for anti-viral therapy comprising a compound of said general formula or functional equivalent thereof, as identified herein. Preferably, said

pharmaceutical composition intended and adapted for anti-viral therapy comprises a compound of said general formula wherein R or R' are as defined above.

Preferably, an anti-viral agent as provided by the invention comprises modifications of 2 acetoxythioanisole, 2-(trifluoromethylacetoxy)thioanisole, 2-($\alpha$ chloroacetoxy)thioanisole, 2-($\alpha$bromoacetoxy)thioanisole, 2-acetoxyphenylbenzyl sulphide, 2-acetoxyphenzyl-2-phenylethyl sulphide, 2-acetoxyphenylethyl sulphide, 2-acetoxyphenylpropyl sulphide, 2-acetoxyphenyl-butyl sulphide, 2-acetoxyphenylpentyl sulphide, 2-acetoxy-phenylhexyl sulphide, 2-acetoxyphenylheptyl sulphide, 2-acetoxyphenyl-2-butoxyethyl sulphide, 2-acetoxyphenyl-2-trans-heptenyl sulphide, 2-acetoxyphenylhept-2-ynyl sulphide, 2-acetoxyphenylbut-2-ynyl sulphide, 2-acetoxyphenylprop-2-ynyl sulphide, or *o*-(acetoxy-phenyl)hept-2-ynyl sulphide (APHS), or a pharmaceutically acceptable salt or hydrate thereof. Suitable pharmaceutically acceptable salts are well known to those skilled in the art and include basic salts of inorganic and organic acids, such as hydrochloric acid, hydrobromic acid, sulphonic acid, phosphoric acid, methane sulphonic acid, ethane sulphonic acid, acetic acid, malic acid, tartaric acid, maleic acid, benzoic acid, salicylic acid, phenylacetic acid and mandelic acid. In addition, pharmaceutically acceptable salts of a compound according to said general formula may also be formed with a pharmaceutically acceptable cation, for instance, if a substituent group comprises a carboxy moiety. Suitable pharmaceutically acceptable cations are well known in the art and include alkaline, alkaline earth ammonium and quaternary ammonium cations. One aim of this study was to investigate whether APHS can act synergistically with the clinically available reverse transcriptase and protease inhibitors (RTI and PI, respectively) *in vitro.* Due to the increasing prevalence of RTI and PI-resistant HIV-1 strains, another aim of this study was to assess the antiviral activity of APHS against drug-resistant HIV-1 strains in vitro. APHS showed synergistic interactions with the RTI zidovudine, lamivudine and efavirenz and with the PI indinavir and ritonavir. The 50% inhibitory concentration ($IC_{50}$) of APHS in this assay dropped from 13 $\mu$M when used alone, to 5 $\mu$M after combination with RTI or PI. In combination with APHS the $IC_{50}$ of the RTI and PI drugs tested also dropped. APHS inhibits the replication of HIV-1 strains resistant to zidovudine, lamivudine, stavudine, didanosine, zalcitabine and ritonavir. The invention provides for example herein that APHS and functional modifications thereof capable of inhibiting a viral polymerase can be combined with RTI and PI and for example can inhibit several NRTI and PI-resistant HIV-1 strains. Modified APHS analogues can for example also be studied and identified by lead-optimalisation protocols such as by determining the effects of said compound's chemical structure in influencing its biological activity, such for example can be determined in assaying quantitative structure-activity relationships (QSAR, Debnath, Mini Reviews in Medicinal chemistry, 2001, 1, 187-195), docking studies and crystallography studies studying the calculated virtual interactions between said modified compounds and a polymerase such as a reverse transcriptase.

Figure legends

Figure 1.1. The effect of APHS on HIV-$1_{Ba\text{-}L}$ replication in PBMC and on PBMC viability. To determine HIV-1 replication APHS was administered simultaneously with HIV-1 and after 7 days HIV-1 production was determined by quantifying p24 antigen concentration in culture supernatant (shown as percentage of the control, which is HIV-infected PBMC not treated with APHS). To determine cellular viability PBMC were incubated with APHS for 7 days after which cellular viability was determined by measuring PBMC metabolic activity (shown as percentage of control, which is PBMC not treated with APHS). Results are representative of ten independent experiments. *$p$<0.05 (according to Student's *t*-test).

Figure 1.2. The effect of APHS or NAC on HIV-1 production by the chronically HIV-1-infected U1 cell line. By incubating the cells with PMA (100 ng/ml) HIV-1 production was stimulated. Afterwards NAC (30 mM) or APHS (3, 10 and 30 $\mu$M) were added and after 2 days HIV-1 production was determined by quantifying p24 antigen concentration (ng/ml) in culture supernatant. Results are the average of three independent experiments. *$p$<0.05 (according to Student's t-test).

Figure 1.3. Number of HIV-$1_{Ba\text{-}L}$ gag RNA copies in PBMC treated with 10 or 30 $\mu$M APHS or 10 or 100 $\mu$g/ml DS as compared to the untreated control 2h after infection. The results of PBMC that were pre-incubated with APHS and DS 24h prior to infection and PBMC that were not pre-incubated are shown. Results are the average of three independent experiments. *$p$<0.05 (according to Student's *t*-test).

Figure 1.4. Number of HIV-$1_{HXB2}$ (-) strong stop (A) and gag (B) DNA copies in PBMC at 10h after infection. PBMC were either untreated (control) or treated with 10 or 30 $\mu$M APHS or 5 or 1000 nM AZT (shown as percentage of the control). Results are the average of three independent experiments. *$p$<0.05 (according to Student's *t*-test).

Figure 1.5. Reverse transcription in the presence of APHS. The DNA primer complementary to the PBS (lanes 1-6) or the natural $tRNA^{lys3}$ primer (lanes 7-12) were heat-annealed onto the *in vitro* synthesized HIV-1 RNA template, and reverse transcription was performed by the addition of dNTPs and HIV-1 RT enzyme (lanes 1 and 7). An increasing amount of APHS (10, 100, 300 and 1000 $\mu$M) was added to the reverse transcription reaction (lanes 2-5 and 8-11). Control reactions in the presence of 10% ethanol were performed in lanes 6 and 12. Results are representative of three independent experiments.

Figure 3.1. Chemical structure of o-(acetoxyphenyl)hept-2-ynyl sulfide (APHS).

Figure 4.1. Effect of APHS and APHS-d on FIV replication in CrFK cells. To determine FIV replication, increasing concentrations of APHS or APHS-d were administered to FIV-infected CrFK cells and after 6 days FIV production was determined by quantifying p24 antigen concentration in culture supernatant (shown as percentage of the control, which is FIV-infected CrFK cells not treated with the compounds). Results represent the average of three independent experiments.

Figure 4.2. Effect of on RSV adhesion to HEp2 cells. HEp2 cells were infected by RSV in the presence of several concentrations APHS for 4, 5 and 6 days. The amount of HEp-2 cells with RSV adherent to their surface (percentage of positive cells) was determined by incubating the cells with a FITC-labeled anti-RSV antibody and measuring the fluorescence by FACS. Results represent the average of three independent experiments.

Detailed description

Example 1 Aspirin-like molecules that inhibit human immunodeficiency virus 1 replication

HIV-1 inhibitors currently used in the clinic target the viral reverse transcriptase (RT) or the viral protease. Treatment of HIV- I -infected individuals with a combination of three or more of these antiretroviral drugs, the so-called highly active antiretroviral therapy (HAART), has proven to be a most successful treatment [1]. Indeed, this strategy has been shown to decrease the HIV-1 viral load, increase the amount of $CD4^+$ lymphocytes and delay disease progression [2,3].
Furthermore, it has several advantages over single drug therapy, like providing an additive or synergistic antiviral effect [4]. Notwithstanding, no drug or combination of drugs is still able to completely block HIV-1 replication or to clear HIV-1 from the long-lived reservoirs of latently infected cells [5]. Furthermore, absence of a proofreading mechanism for the RT allows emergence of $4 \times 10^{-5}$ mutations per base pair per cycle [6]. Since the estimated average of HIV-1 production is $10.5 \times 10^9$ virions per day [7], replication-competent HAART-resistant viruses will eventually be selected. Also, long-term toxicity of current drugs and poor adherence to the treatment are important causes of therapy failure [8]. Therefore, new medicines and strategies are needed to eliminate HIV. Besides more effective and less toxic new RT inhibitors (RTI) and protease inhibitors (PI) several new compounds that target viral entry, virus-cell fusion, viral assembly and disassembly, proviral DNA integration and viral mRNA transcription are in development [9]. Cellular factors involved in HIV entry (like CD4, CCR5 and CXCR4) and HIV transcription (such as NF-κB and Sp1) have also been described as targets for anti-HIV therapy [10]. The anti-proliferation drug hydroxyurea also possesses anti-HIV activity *in-vitro*[11]. Since it is known that inflammatory molecules such as cytokines and oxygen radicals are involved in the pathogenesis of HIV-1 infection, many anti-inflammatory compounds including platelet-activating factor receptor antagonists, aspirin and indomethacin have shown to possess anti-HIV potential [12-14]. o-(acetoxyphenyl)hept-2-ynyl sulfide (APHS) is an aspirin-like molecule that belongs to the group of selective nonsteroidal anti-inflammatory drugs (NSAIDs) [15]. This new generation of selective NSAIDs preferentially acetylates and irreversibly inactivates COX-2 and maintains a good anti-inflammatory activity. Moreover, concentrations of APHS up to 100 mg/kg showed no toxicity in the rat air pouch model [15].
The aim of this study was to determine whether APHS possesses anti-HIV-1 activity and, if so, by which mechanism.

Materials and Methods

*Isolation of primary cells*

Donor peripheral blood mononuclear cells (PBMC) were isolated from heparinized blood from HIV-1-, HIV-2-, and hepatitis B-seronegative donors and obtained on Ficoll-Isopaque density gradients. To prepare a PBMC mixed batch, PBMC isolated from six donors were pooled together in RPMI 1640 medium (Gibco, Invitrogen, Paisley, Scotland) supplemented with 10% dimethyl sulfoxide (DMSO; Merck, Darmstadt, Germany), 20% fetal calf serum (FCS; Invitrogen) and 10 μg/ml gentamycin (Invitrogen) and frozen at -140°C. Cells were thawed and cultured for 4 days prior to the experiment in RPMI 1640 medium supplemented with 10% FCS, 10 μg/ml gentamycin and 2 μg/ml lectin from *Phaseolus vulgaris* (PHA, Sigma Chemie, Zwijndrecht, The Netherlands) at 37 °C and 5% $CO_2$.
Peripheral blood lymphocytes (PBL) were isolated after incubation of PBMC in tissue culture flasks for 1 hour. Monocytes adhered to the flask and PBL-containing supernatant was collected, seeded in culture flasks with medium (RPMI 1640 supplemented with 10% FCS and 10 μg/ml gentamycin) at a concentration of $2 \times 10^6$ cells/ml, stimulated with 2 μg/ml PHA and incubated for 4 days at 37 °C and 5% $CO_2$.
To obtain monocytes, PBMC were washed twice and monocytes were purified by countercurrent centrifugal elutriation. Cells were >98% monocytes by criteria of cell morphology on May-Grünwald-Giemsa-stained cytosmears and by non-specific esterase staining using α-naphtylacetate (Sigma) as substrate. Monocytes were cultured in suspension at a

concentration of 2 x $10^6$ cells/ml in Teflon flasks (Nalgene, Rochester, NY) in Dulbecco's modified Eagle's medium (DMEM) with 10% heat-inactivated human AB serum negative for anti-HIV antibodies, 10 µg/ml gentamycin, and 10 µg/ml ciprofloxacin (Sigma) for 7 days at 37 °C and 5% $CO_2$. As previously described, HIV-1 infection of non-adherent macrophages, especially when using a low multiplicity of infection, appears much more reproducible than infection of macrophages that were first allowed to adhere [16].

*Compounds*

APHS and derivatives were supplied by Dr. L.J. Marnett. Synthesis details have been described by Kalgutkar and colleagues [17]. All compounds tested were diluted in aliquots in 100% ethanol, topped with argon gas and stored at -20 °C. The concentration of ethanol during incubations never exceeded 0.1%. At this concentration, ethanol did not affect HIV-1 replication or cellular viability (data not shown). Zidovudine (AZT; Sigma) was diluted in DMSO. The concentration of DMSO during incubations never exceeded 0.001% and no effect on HIV-1 replication or cellular viability was observed at this concentration (data not shown). Dextran sulphate (DS; Sigma) was diluted in 100% ethanol. The concentration of ethanol during incubations with DS never exceeded 0.1%.

*HIV- 1 infection of primary cells*

Monocyte-derived macrophages (MDMs) and PHA-stimulated PBL or PBMC were washed twice and incubated for 7 days at a density of $5 \times 10^5$ cells/ml with HIV-$1_{Ba\text{-}L}$, HIV-$1_{AT}$ or HIV-$1_{HXB2}$ at a multiplicity of infection (M. O.I.) of 0.0025 (input virus), in the presence of AZT, APHS or related compounds and 10 U/ml recombinant IL-2 (Roche Diagnostics GmbH, Mannheim, Germany), at 37 °C and 5% $CO_2$. To correct for the input virus, an extra control consisting of medium containing the same amount of input virus as added to the cells was included in the experiment. The amount of p24 in this control was subsequently subtracted from the p24 values of the samples in order to obtain the exact amount of p24 produced by the cells. *p24-core antigen quantification by ELISA*
After 2 or 7 days incubation, samples of the supernatants were collected, inactivated by addition of Empigen (Calbiochem, La Jolla, CA, USA) and by heat inactivation at 56°C for 30 min. p24-core antigen concentration was determined by an enzyme linked immunoabsorbent assay (ELISA) (AMPAK™, DAKO, Cambridgeshire, UK) as described previously [18,19]. The optical density values were converted into p24 concentration (ng/ml) with the use of a calibration curve made by serial dilutions of recombinant p24 protein (NIBSC, UK) that was submitted to the same treatment as the samples.

*Determination of viability of primary cells*

MDM and PHA-stimulated PBMC or PBL were washed twice and incubated at a density of $5 \times 10^5$ cells/ml with increasing concentrations of AZT, APHS or related compounds for 7 days at 37 °C and 5% $CO_2$ Afterwards the metabolic activity of these cells was assessed by a cellular viability assay as described previously [20].
Shortly, 150 µg/ml tetrazollium salt 3-(4,5-dimethylthiazol-2-yl)-2,5-diphenyltetrazolliumbromide (MTT; Sigma) was added to the cells. During the subsequent 2h incubation period metabolic active cells converted MTT into blue formazan crystals. Afterwards, ¾ of the supernantant was gently removed and substituted by stop buffer consisting of 90% 2-propanol, 10% Triton X-100 and 0.4% HCl (Merck, Darmstadt, Germany). When all formazan crystals were dissolved optical density was measured at 550 nm and using 630 nm as reference.

*Infection model with the monocytic cell line U1*

U1 cells (AIDS Reagents, Rockville, MA) were incubated with 100 ng/ml phorbol myristate acetate (PMA; Sigma) or with medium (negative control) for 30 min. Afterwards the cells were washed and seeded in a concentration of $1 \times 10^6$ cells/ml in RPMI 1640 medium containing 10% FCS and 10 µg/ml gentamycin either with increasing concentrations of APHS or with 30 mM N-acetyl-L-cysteine (NAC; Sigma), which served as a positive control. After 2 days of incubation, samples of the supernatant were collected, inactivated by addition of Empigen and by 30 min incubation at 56°C. The concentration of p24 was determined by ELISA (AMPAK™) as described above.

*Determination of $IC_{50}$ and $TC_{50}$ of the compounds*

The ability of each compound to decrease p24 antigen and cellular viability were expressed as the 50% inhibitory concentration ($IC_{50}$) and the 50% toxic concentration ($TC_{50}$), respectively. $IC_{50}$ and $TC_{50}$ were calculated using the computer software program CalcuSyn for Windows (Biosoft, Cambridge, UK) according to the method of Chou and Talalay [21,21]. This program uses the median-effect equation to produce dose-effect curves:

$$f_a = 1/[1 + (D_m/D)^m] \quad \text{[equation 1]}$$

where $f_a$ represents fraction affected by the dose (reduction of p24 or cellular viability at a certain drug concentration expressed in decimals), $D_m$ is the median effect dose (same as $IC_{50}$ or $TC_{50}$), $D$ is the dose of the drug and $m$ is the sigmoidicity coefficient of the dose-effect curve. Data was accepted when the linear correlation coefficient of the median-effect plot based on experimental data was > 0.90. The selectivity index (SI), which represents the concentration range where a drug is effective without being toxic, was calculated by the ratio $TC_{50}/IC_{50}$.

*PBMC infection with HIV-1$_{Ba\text{-}L}$ or HIV-1$_{HXB2}$ for* real-time *Taqman PCR* The ability of HIV-1$_{Ba\text{-}L}$ to enter the cells in the presence of APHS was tested using DS as positive control (entry assay). The amount of HIV-1$_{HXB2}$ reverse transcription products in the presence of APHS was also tested, using AZT as positive control (reverse transcription assay).

Before infection, PHA-stimulated PBMC from the PBMC batch were pre-incubated with different concentrations of APHS, DS or AZT for 24 h. Afterwards, cells were washed and $1 \times 10^6$ PBMC were incubated for 40 min with HIV-1$_{Ba\text{-}L}$ or HIV-1$_{HXB2}$ at an M.O.I. of 0.0025 and with appropriate concentrations of APHS, AZT, DS or medium in the presence of 1/20 concentrated M buffer (Roche) and 4U/ml DNase (Roche) to digest possible contaminating viral DNA associated with the virions.

Afterwards PBMC were washed and resuspended in medium with or without compounds. Cells were treated with 1 mg/ml Pronase (Roche) to remove virus particles bound to the cells. After 5 minutes of incubation at 37°C, 5% $CO_2$, the cells were washed three times, resuspended in medium with or without the compounds and incubated at 37°C and 5 % $CO_2$. At different time points, $1.0 \times 10^6$ cells were added to 900 µl Nuclisens lysis buffer (Organon Teknika, Boxtel, The Netherlands) containing 40 µl silica to bind total nucleic acids (Nuclisens isolation kit). For the entry assay, samples were collected 2h after the start of the infection and for the reverse transcription assays samples were collected 2, 7 and 10h after the start of the infection.

*HIV RNA/DNA extraction*

HIV RNA and DNA were extracted from samples of infected cells according to a method described before [23]. Briefly, after 10 min of mixing the silica and lysis buffer, tubes were shortly centrifuged at 13,000 rpm and supernatant was discarded. Free non-nucleic acid components were removed by washing the silica particles twice with Nuclisens washing buffer, twice with 70 % ethanol and once with acetone. The silica pellet was dried for 10 min at 56 °C. Total nucleic acids were eluted by resuspending the pellet in 100 µl nuclease-free water. After mixing the tubes for 10 min at 56 °C, tubes were shortly centrifuged and the supernatant was transferred to a new tube and stored at -20 °C.

*RT-PCR using random hexamers*

Extracted RNA was subjected to reverse transcriptase-polymerase chain reaction (RT-PCR) to obtain cDNA. The PCR mixture consisted of 10 µl of extracted RNA, 1× RT buffer, 5.5 mM $MgCl_2$, 0.5 mM dNTP, 2.5 µM Random hexameres, 0.4 U/µl RNase inhibitor, 1.25 U/µl MultiScribe RT (Taqman reverse transcription reagents kit, Applied Biosystems, Forster City, CA, USA) and nuclease-free water.

Amplification was performed in a GeneAmp PCR system 2400 (Perkin-Elmer) with a cycle program of 10 min at 25 °C, 30 min at 48 °C and 5 min at 95 °C.

*Real-time 5' nuclease (Taqman) PCR*

Real-time Taqman PCR is based on cleavage of a probe, hybridized to the target sequence by 5'-3' nuclease activity of Taq DNA polymerase during amplification, as described previously [24]. Briefly, target sequence-specific forward and reverse primers prime the amplification reaction. The probe contains a reporter dye at the 5' end and a quencher dye at the 3' end. When both dyes are attached to the probe, reporter dye emission is quenched. The probe is displaced from the target during each polymeration cycle. Once separated from the quencher, the reporter dye emits its characteristic fluorescence, which can then be measured. The amount of fluorescence measured is proportional to the amount of PCR product.

HIV-1 gag cDNA and HIV-1 (-) strong stop and gag DNA were amplified and quantified by real-time Taqman PCR as described elsewhere [25]. Sets of primers/probes were designed according to a previous study by Zack and colleagues [26]. To quantify the amount of gag RNA in the samples, a cDNA standard curve was prepared from five-fold dilutions of extracted RNA from an HXB2 viral batch, of which the RNA amount was previously determined. To quantify (-) strong stop and gag DNA in the samples, a DNA standard curve was prepared from five-fold dilutions of DNA

extracted from U1 cells, which contain two copies of the HIV-$1_{HXB2}$ genome per cell. Albumin was also quantified and used for normalization of all samples.

*Cell-free RT assay*

The HIV-1 RNA template (positions +1 to +368, with +1 being the capped G residue) was synthesized *in vitro* with T7 RNA polymerase as described previously [27]. In the reverse transcription assay, 10 ng of the RNA template was incubated with 1.5 μg calf liver tRNA (6 pmol total tRNA, of which approximately 1.2 pmol $tRNA^{lys3}$, Boehringer) or 20 ng of a DNA primer complementary to the primer binding site (PBS) (positions +182 to +202) in 12 μl annealing buffer (83 mM Tris-HCl pH 7.5, 125 mM KCl) at 85 °C for 2 min and 65 °C for 10 min, followed by cooling to room temperature over a 1h period. Reverse transcription was initiated by the addition of 6 μl RT buffer (9 mM $MgCl_2$, 30 mM DTT, 150 μg/ml actinomycine D, 30 μM dATP, dGTP and dTTP and 1.5 μM dCTP), 0.5 μl [$\alpha$-$^{32}$P]dCTP and 0.5 U HIV-1 RT (MRC) in the presence or absence of APHS (10, 30, 300 and 1000 μM) or 10% ethanol. The reaction was incubated for 30 min at 37 °C. The cDNA product was precipitated in 0.3 M sodiumacetate, pH 5.2 and 80% ethanol at-20°C, dissolved in formamide loading buffer and analyzed on a denaturing 6% polyacrylamide-urea sequencing gel.

Results

*Effect of APHS on HIV-1 replication in primary cells*

To assess whether APHS was capable of inhibiting HIV-1 replication, HIV-1 infection of PBMC was performed as described above. HIV-1 production was assessed by quantifying the levels of the HIV-1 capsid protein p24 in culture supernatants. The $IC_{50}$ of AZT in PBMC was 3 nM (data not shown). This value fell within the reported range published in the Physicians' Desk Reference 2000 [28].
APHS is capable of inhibiting HIV-$1_{Ba\text{-}L}$ production in PBMC in a dose-dependent manner (Fig. 1.1.), while mock-infected cells showed undetectable levels of p24 (data not shown). 30 μM APHS inhibit HIV-$1_{Ba\text{-}L}$ replication by 80%. When the M.O.I. was increased from 0.0025 to 0.01, the $IC_{50}$ increased by a factor of 1.4 (data not shown).
APHS is also capable of inhibiting HIV-1 replication in MDM and PBL in a dose-dependent manner. Results are shown in Table 1. The $IC_{50}$ of APHS in PBMC infected with HIV-$1_{Ba\text{-}L}$, HIV-$1_{AT}$ or HIV-$1_{HXB2}$ was 6, 7 and 11 μM, respectively. The $IC_{50}$ of APHS in MDM infected with HIV-$1_{Ba\text{-}L}$ and in PBL infected with HIV-$1_{HXB2}$ was 12 μM.

*Effect of APHS on viability of primary cells*

To determine whether APHS could affect cellular viability, a cellular viability assay was performed as described above. The cytotoxicity of APHS was determined by quantifying cellular metabolic activity. The $TC_{50}$ of AZT in PBMC was higher than 80 μM (data not shown). This value fell within the reported range published in the Physicians' Desk Reference 2000 [28]. The effect of APHS on PBMC viability is shown in Fig. 1.1. APHS was cytotoxic at concentrations above 30 μM. Table 1.1 shows the effect of APHS in PBMC, MDM and PBL. The $TC_{50}$ of APHS in PBMC, MDM and PBL were 105, 235 and 147 μM, respectively.

*Activity of APHS and related compounds*

Since APHS was designed to be a potent and selective COX-2 inhibitor, a possible correlation between HIV-1 inhibition capacity and COX-2 inhibition capacity was studied further by using several compounds related to APHS, with and without COX-2 activity (Table 1.2). The following compounds are not clinically available: APHS-d1, a sulfone derivative of APHS without anti-COX-2 activity; APHS-d2, a heptyl sulfide derivative of APHS which is as potent as APHS but less selective against COX-2 than APHS; APHS-d3, a phenol hydrolysis product of APHS without COX-2 activity and APHS-d4, a methylsulfide analog of APHS which is more selective than APHS but 100-fold less potent. Also commercially available COX-2 inhibitors were tested: aspirin, which is 60 times less potent and 100 times less selective than APHS; indomethacin, which is as potent but less selective than APHS and ibuprofen, which is less potent and less selective than APHS. The $IC_{50}$ and $TC_{50}$ of these compounds were determined in PBMC and the results are shown in Table 1.2. APHS and compounds APHS-d1 and APHS-d2 showed the highest SI ratios (18, 86 and 41, respectively). Compounds APHS-d3 and APHS-d4 showed lower SI ratios (lower than 0.1 and 2, respectively). Interestingly, aspirin could also inhibit HIV-1 replication but with an $IC_{50}$ of approximately 100 times higher than APHS and a SI of 17. Indomethacin and ibuprofen showed SI values of 3 and 5, respectively. Since compounds APHS-d1 and APHS-d3 are not COX-2 inhibitors and since the clinically used COX-2 inhibitors tested did not show good anti-HIV activity, the anti-HIV-1 activity of APHS does not seem to be related with its COX-2 inhibition activity.

*Effect of APHS on HIV-1 production in U1 cells*

To further elucidate the mechanism of action of APHS, a chronic infection model consisting of U1 cells that were incubated with increasing concentrations of APHS was performed. U1 is a clone of the U937 cell line, which is chronically infected with HIV-1 and shows minimal constitutive expression of HIV-1. When stimulated with cytokines or PMA, the production of HIV-1 by these cells increases 20-fold. HIV-1 production was assessed by quantifying the levels of the HIV-1 capsid protein p24 in culture supernatants of PMA-treated cells (Fig. 1.2). NAC was used as a positive control because it can block the PMA-induced HIV-1 expression. APHS was not capable of inhibiting HIV-1 production in this model. This supports the fact that APHS cannot inhibit the post-integration steps of the HIV-1 life cycle.

*Effect of APHS on HIV-1 entry into target cells*

Since APHS seems to work at a step prior to transcription, a series of experiments was performed in order to determine the exact point in the HIV-1 life cycle that is being inhibited by APHS. The first set of experiments consisted in testing the effect of APHS on the entry of HIV-1 virions into PBMC. The amount of gag cDNA, which represents the viral RNA that entered into the target cells, was quantified. DS, a known entry inhibitor [29], was used as a positive control. The entry of HIV-1 virions was also investigated in PBMC that were pre-incubated with APHS and DS 24h prior to infection. None of the drug concentrations used were cytotoxic to PBMC after 24h incubation. Similarly to what was observed in the cytotoxic assay (Fig. 1.1.), APHS concentrations above 30 μM proved to be significantly cytotoxic when compared to untreated cells 24h after incubation and were therefore not included in this assay. To measure the amount of HIV-1 Ba-L RNA that has entered the cells 2h after infection, viral gag RNA was quantified by real-time Taqman PCR. The results are shown in Fig. 1.3. DS inhibited viral entry significantly by approximately 80% at all concentrations used ($p<0.04$). The inhibition of viral entry by DS was further improved when PBMC were first pre-incubated with the compound. PBMC that were treated with APHS showed no inhibition of viral entry. Also, the amount of viral RNA copies was not significantly decreased, when PBMC were pre-incubated with APHS prior to infection.

*Effect of APHS on cellular HIV-1 reverse transcription*

The effect of APHS on reverse transcription was also investigated using the RTI AZT as a positive control. None of the drug at the concentrations used were cytotoxic to PBMC after 24h incubation (not shown). PBMC were pre-incubated for 24h with different concentrations of APHS and AZT. HIV-$1_{HXB2}$ DNA extracted from these cells was subjected to real-time Taqman PCR in order to quantify different parts of the HIV-1 genome which represent different stages of reverse transcription: (-) strong stop DNA is produced before the first template switch and gag DNA is produced after the first template switch.

The effect of APHS and AZT on the synthesis of (-) strong stop and gag DNA on completion of reverse transcription are shown in Fig. 1.4. Since in our assay, reverse transcription was completed between 7 and 10h the results at 10h are shown here. APHS- or AZT-treated PBMC did not show a significant decrease in (-) strong stop DNA copy numbers as compared to control untreated PBMC (Fig. 1.4A). It has previously been reported that (-) strong-stop DNA production is relatively insensitive to AZT. AZT preferentially terminates viral DNA synthesis after the first template switch and in this way (-) strong-stop DNA can be present for at least 6 days after infection in AZT-treated cultures. [30]. It is also known that partial reverse transcription is initiated in the virion before entry into the host cell resulting in an excess of early versus late transcripts inside the virion [31]. A small but not significant increase in (-) strong-stop DNA copies was observed for PBMC treated with 30μM APHS and 5nM AZT.

The gag cDNA levels after completion of reverse transcription in APHS- or AZT-treated PBMC as compared to untreated PBMC is shown in Fig. 1.4B. At 10h after infection, no significant differences could be detected between untreated PBMC and PBMC treated with 10 μM of APHS. However, 30 μM APHS significantly inhibited gag production by 60%. A 35% or 90% reduction in gag cDNA copies was observed in PBMC treated with 5 and 1000 nM AZT, respectively.

*Cell-free RT assay*

To test whether APHS can inhibit RT directly, we performed a cell-free RT assay. We used an *in vitro* synthesized HIV-1 RNA template encompassing the complete untranslated leader region (+1 to +368) in combination either with the natural $tRNA^{lys3}$ primer or with a DNA primer complementary to the PBS to initiate reverse transcription (Fig. 1.5). The primers were heat-annealed at 85 °C, and reverse transcription was initiated by the addition of dNTPs and HIV-1 RT enzyme. Extension of the DNA primer produces a full-length cDNA product of 202 nt (lane 1), whereas extension of the tRNA primer produces a 257 nt product (lane 7).

Shorter cDNAs represent RT pauses due to stable RNA secondary structure in the HIV-1 template [32]. Concentrations of APHS that ranged from 10 to 300 μM did not affect cDNA formation, while 1000 μM APHS decreased

the amount of reverse transcription product. However, addition of APHS from the stock solution in ethanol raised the ethanol concentration during reverse transcription to 5% for the 1000 μM sample. Therefore, control reactions were performed in the presence of 10% ethanol (lanes 6 and 12). Indeed, ethanol by itself inhibited the reverse transcription. This indicates that the effect observed with 1000 μM APHS is probably due to the ethanol in the sample.

In this example 1 it was demonstrated that the new NSAID APHS could inhibit the replication of several HIV-1 strains in human primary cells (PBMC, MDM and PBL) at non-toxic concentrations. Since it was previously shown that cell proliferation is not required for productive HIV-1 infection of MDM [33], it can be concluded that the antiviral activity of APHS is not related with inhibition of proliferation. The other NSAIDs tested did not show better anti-HIV activity than APHS, which suggests that anti-inflammatory activity is not necessary for anti-HIV activity. Furthermore, one of two APHS derivatives that had no anti-inflammatory activity did inhibit HIV-1 replication better than APHS did. APHS did inhibit HIV-1 replication before provirus integration into the host cell genome occurred. HIV-1 life cycle kinetics was then used to investigate the possible effect(s) of APHS on entry of HIV-1 into host cells and on reverse transcription. It has been estimated that all virions, bound to the host cell, will have entered the cell 2 hours after infection [34]. In the entry assay, entry of HIV-1 virions was measured at 2h after infection. DS, which inhibits binding of virions to the host cell by interacting with gp 120 [29], significantly decreased the amount of viral gag RNA entering the host cell. APHS did not have a significant effect on entry of HIV-1 virions into host cells.

The RTI AZT significantly decreased the amount of gag DNA but not of (-) strong stop DNA, which is produced before the first template switch. Indeed, previous studies have shown that AZT is preferentially incorporated into the growing DNA chain after the first template switch [30]. At 30 μM APHS was able to decrease the production of gag DNA, which is a late product of the reverse transcription. This observation suggests that APHS may target a step between entry and reverse transcription or the reverse transcription itself. But since APHS does not inhibit the production of (-) strong stop DNA, which is an early product of the reverse transcription, it can be concluded that APHS is targeting the reverse transcription itself. Surprisingly, at 10 μM, the $IC_{50}$ of APHS in primary cells as described previously, APHS did not inhibit gag DNA production in the real-time Taqman PCR assay. This discrepancy is probably due to experimental differences between the two assays. Perhaps longer incubation times may enable APHS to protect more cells.

Also the method used for quantification of HIV replication differs between the two assays. The same phenomenon was observed for AZT. The IC50 of AZT in the PBMC was 3 nM while in the real-time Taqman PCR 5 nM showed only 40% protection against HIV. The effect of APHS could not be studied at concentration higher than 30 μM because of toxicity for the cells. Compounds are being developed by systemic variation of structural groups of APHS in order to find a compound related to APHS, which may have a more favorable selectivity index. Thus, APHS is inhibiting HIV-1 replication by interfering with the RT enzyme or with some viral or cellular factor(s) involved in the reverse transcription process.

The most critical cellular factor involved in reverse transcription is the deoxynucleoside-5'-triphosphate (dNTP) pool. APHS could have a mechanism of action similar to hydroxyurea, which is known to inhibit the cellular enzyme ribonucleotide reductase, thus reducing the intracellular levels of dNTPs and, in this way, impeding HIV DNA synthesis [11]. It is also known that the virus requires no cellular factors after infection, except for a nucleotide pool, to achieve initiation of reverse transcription [35]. Since we know that APHS could not affect the replication of the first product of the reverse transcription, it is reasonable to assume that APHS is not affecting dNTP levels in the cell.

Many viral factors including nef, tat, vif, vpr, matrix protein, nucleocapsid protein and integrase are involved in reverse transcription [35] and are potential targets for APHS. APHS can inhibit the production of the reverse transcription gag DNA but not of the (-) strong stop DNA indicating that APHS inhibits the elongation process and not the initiation of reverse transcription. This mechanism of action is similar to that of nucleoside RT inhibitors (NRTI) but not to that of non-nucleoside RT inhibitors (NNRTI) [36]. Moreover, the structure of APHS is very different from a typical NRTI. Interestingly, a previous study demonstrated the potential of aspirin in the synthesis of AZT and 3'-deoxy-3'-fluorothymidine (FLT) prodrugs [37]. Our findings suggest that APHS may target reverse transcription in a different way from that of the known RTI.

APHS showed no inhibition of RT activity in the primer extension assay. This result can be interpreted in two different ways: APHS does not inhibit RT directly but it affects some cellular or viral factor involved in reverse transcription process or APHS may have to be metabolized by the cell in order to become active and, in this way, its anti-RT activity can only be detected in cell-based systems. This last option is reinforced by the fact that the NRTIs AZT and dideoxyinosine (ddI) have to be phosphorylated by cellular enzymes to become active. These NRTIs are only able to terminate chain elongation in cell-free assays when added in the triphosphate form (AZT-TP, ddITP) [38]. We do not yet know how the cell metabolizes APHS into its antivirally active form.

The fact that APHS is also an anti-inflammatory compound may be useful in anti-HIV therapy because it targets not only virus replication but also inflammatory processes. This can be beneficial in reaching areas like the brain where inflammation is thought to account for severe neurodegeneration associated with HIV-1 infection [39].

Table 1.1

| The effect of APHS on cellular viability and HIV-$1_{Ba-L}$, HIV-$1_{HXB2}$, HIV-$1_{AT}$ replication in PBMC, MDM and PBL. | | | |
|---|---|---|---|
| Cells | HIV-1 strain | $IC_{50}$[a](μM) | $TC_{50}$[b](μM) |
| PBMC | Ba-L | 6 ± 1 | 105 ± 15 |
| | AT | 7 ± 5 | |
| | HXB2 | 11±7 | |
| MDM | Ba-L | 12±4 | 235±127 |
| PBL | HXB2 | 12±4 | 147±71 |

[a]$IC_{50}$ represents 50% inhibitory concentration.
[b] $TC_{50}$ represents 50% toxic concentration.

Results are the average of ten independent experiments.

Table 1.2 Effect of APHS, four APHS derivatives, aspirin, indomethacin and ibuprofen on HIV-$1_{Ba\text{-}L}$ replication and cellular viability in PBMC. Chemical structure of APHS, APHS derivatives and aspirin are also shown.

| | $X_1$ | $X_2$ | $R_1$ | $R_2$ | $R_3$ | $IC_{50}$[a] (HIV-1) | $TC_{50}$[b] (HIV-1) | SI[c] (HIV-1) |
|---|---|---|---|---|---|---|---|---|
| APHS | S | C | $CH_2C\equiv C(CH_2)_3CH_3$ | $CH_3$ | O | 6 ± 1 | 105 ± 15 | 18 |
| APHS -d1 | $SO_2$ | C | $CH_2C\equiv C(CH_2)_3CH_3$ | $CH_3$ | O | 4 ± 1 | 345 ± 131 | 86 |
| APHS -d2 | S | C | $(CH_2)_6CH_3$ | $CH_3$ | O | 5 ± 1 | 205 ± 168 | 41 |
| APHS -d3 | S | H | $CH_2C\equiv C(CH_2)_3CH_3$ | — | — | 28 ± 4 | 3 ± 1 | <0 |
| APHS -d4 | S | C | $CH_3$ | $CH_3$ | O | 223 ± 15 | 340 ± 151 | 2 |
| Aspirin | — | C | $CO_2H$ | $CH_3$ | O | 527 ± 210 | 8977 ± 8486 | 17 |
| Indomethacin | | | | | | 21 ± 2 | 71 ± 10 | 3 |
| Ibuprofen | | | | | | 44 ± 37 | 219 ± 41 | 5 |

[a] $IC_{50}$ represents 50% inhibitory concentration.

[b] $TC_{50}$ represents 50% toxic concentration.

[c] The SI represents the $TC_{50}$ / $IC_{50}$ ratio.

Results are the average of ten independent experiments.

References with example 1

1. Hammer,S.M., Kessler,H.A. & Saag,M.S. Issues in combination antiretroviral therapy: a review. *J. Acquir. Immune. Defic. Syndr.* 7 Suppl 2, S24-S35 (1994).

2. Palella,F.J., Jr. *et al.* Declining morbidity and mortality among patients with advanced human immunodeficiency virus infection. HIV Outpatient Study Investigators. *N. Engl. J. Med.* 338, 853-860 (1998).

3. Henry,K. *et al.* A randomized, controlled, double-blind study comparing the survival benefit of four different reverse transcriptase inhibitor therapies (three-drug, two-drug, and alternating drug) for the treatment of advanced AIDS. AIDS Clinical Trial Group 193A Study Team. *J. Acquir. Immune. Defic. Syndr. Hum. Retrovirol.* 19, 339-349 (1998).

4. Snyder,S., D'Argenio,D.Z., Weislow,O., Bilello,J.A. & Drusano,G.L. The triple combination indinavir-zidovudine-lamivudine is highly synergistic. *Antimicrob. Agents Chemother.* 44, 1051-1058 (2000).

5. Ramratnam,B. *et al.* The decay of the latent reservoir of replication-competent HIV-1 is inversely correlated with the extent of residual viral replication during prolonged anti-retroviral therapy. *Nat. Med.* 6, 82-85 (2000).

6. Mansky,L.M. The mutation rate of human immunodeficiency virus type 1 is influenced by the vpr gene. Virology 222, 391-400 (1996).

7. Perelson,A.S., Neumann,A.U., Markowitz,M., Leonard,J.M. & Ho,D.D. HIV-1 dynamics in vivo: virion clearance rate, infected cell life-span, and viral generation time. Science 271, 1582-1586 (1996).

8. Max,B. & Sherer,R. Management of the adverse effects of antiretroviral therapy and medication adherence. *Clin. Infect Dis.* 30 Suppl 2, S96-116 (2000).

9. De Clercq,E. Novel compounds in preclinical/early clinical development for the treatment of HIV infections. *Rev. Med. Virol.* 10, 255-277 (2000).

10. Baba,M. Cellular factors as alternative targets for inhibition of HIV-1. *Antiviral Res.* 33, 141-152 (1997).

11. Gao,W.Y., Cara,A., Gallo,R.C. & Lori,F. Low levels of deoxynucleotides in peripheral blood lymphocytes: a strategy to inhibit human immunodeficiency virus type 1 replication. *Proc. Natl. Acad. Sci. U. S.* A 90, 8925-8928 (1993).

12. Kopp,E. & Ghosh,S. Inhibition of NF-kappa B by sodium salicylate and aspirin. *Science* 265 , 956-959 (1994).

13. Serradji,N. *et al.* Structure-activity relationships in platelet-activating factor (PAF). 10. From PAF antagonism to inhibition of HIV-1 replication. *J. Med. Chem.* 43, 2149-2154 (2000).

14. Bourinbaiar,A.S. & Lee-Huang,S. The non-steroidal anti-inflammatory drug, indomethacin, as an inhibitor of HIV replication. *FEBS Lett.* 360, 85-88 (1995).

15. Kalgutkar,A.S. *et al.* Aspirin-like molecules that covalently inactivate cyclooxygenase-2. *Science* 280, 1268-1270 (1998).

16. Boven,L.A. *et al.* Increased peroxynitrite activity in AIDS dementia complex: implications for the neuropathogenesis of HIV-1 infection. *J. Immunol.* 162, 4319-4327 (1999).

17. Kalgutkar,A.S., Kozak,K.R., Crews,B.C., Hochgesang,G.P.J. & Marnett,L.J. Covalent modification of cyclooxygenase-2 (COX-2) by 2-acetoxyphenyl alkyl sulfides, a new class of selective COX-2 inactivators. *J. Med. Chem.* 41, 4800-4818 (1998).

18. McKeating,J.A., McKnight,A. & Moore,J.P. Differential loss of envelope glycoprotein gp 120 from virions of

human immunodeficiency virus type 1 isolates: effects on infectivity and neutralization. *J. Virol.* 65, 852-860 (1991).

19. Moore,J.P., McKeating,J.A., Weiss,R.A. & Sattentau,Q.J. Dissociation of gp120 from HIV-1 virions induced by soluble CD4. *Science* 250, 1139-1142 (1990).

20. Pauwels,R. *et al.* Rapid and automated tetrazolium-based colorimetric assay for the detection of anti-HIV compounds. *J. Virol. Methods* 20, 309-321 (1988).

21. Chou,T.C. & Talalay,P. Quantitative analysis of dose-effect relationships: the combined effects of multiple drugs or enzyme inhibitors. *Adv. Enzyme Regul.* 22, 27-55 (1984).

22. Chou,T.C. & Hayball,M.P. CalcuSyn: Windows software for Dose Effect Analysis. (BIOSOFT, Cambridge,UK, 1996).

23. Boom,R. *et al.* Rapid and simple method for purification of nucleic acids. *J. Clin. Microbiol.* 28, 495-503 (1990).

24. Holland,P.M., Abramson,R.D., Watson,R. & Gelfand,D.H. Detection of specific polymerase chain reaction product by utilizing the 5'----3' exonuclease activity of Thermus aquaticus DNA polymerase. *Proc. Natl. Acad. Sci. U. S. A* 88, 7276-7280 (1991).

25. Pereira,C.F., Nijhuis,M., de Graaf,L., den Dunnen, J., Wieffer, M., Boucher, C.A.B., Schuurman, R.. Novel Assay for Fitness Analysis of Drug-Resistant Human Immunodeficiency Virus-1. *submitted for publication.*

26. Zack,J.A. *et al.* HIV-1 entry into quiescent primary lymphocytes: molecular analysis reveals a labile, latent viral structure. *Cell* 61, 213-222 (1990).

27. Beerens,N. & Berkhout,B. In vitro studies on tRNA annealing and reverse transcription with mutant HIV-1 RNA templates. *J. Biol. Chem.* 275, 15474-15481 (2000).

28. Physician's Desk Reference. PDR Medical Economics Company, Montvale, NJ (2000).

29. Este,J.A. *et al.* Development of resistance of human immunodeficiency virus type 1 to dextran sulfate associated with the emergence of specific mutations in the envelope gp120 glycoprotein. *Mol. Pharmacol.* 52, 98-104 (1997).

30. Arts,E.J. & Wainberg,M.A. Preferential incorporation of nucleoside analogs after template switching during human immunodeficiency virus reverse transcription. *Antimicrob. Agents Chemother.* 38, 1008-1016 (1994).

31. Trono,D. Partial reverse transcripts in virions from human immunodeficiency and murine leukemia viruses. *J. Virol.* 66, 4893-4900 (1992).

32. Beerens,N., Groot,F. & Berkhout,B. Stabilization of the U5-leader stem in the HIV-1 RNA genome affects initiation and elongation of reverse transcription. *Nucleic Acids Res.* 28, 4130-4137 (2000).

33. Schmidtmayerova,H., Nuovo,G.J. & Bukrinsky,M. Cell proliferation is not required for productive HIV-1 infection of macrophages. *Virology* 232, 379-384 (1997).

34. Reddy,B. & Yin,J. Quantitative intracellular kinetics of HIV type 1. *AIDS Res. Hum. Retroviruses* 15, 273-283 (1999).

35. Harrich,D. & Hooker,B. Mechanistic aspects of HIV-1 reverse transcription initiation. *Rev. Med. Virol.* 12, 31-45 (2002).

36. Hooker,C.W., Lott,W.B. & Harrich,D. Inhibitors of human immunodeficiency virus type 1 reverse transcriptase target distinct phases of early reverse transcription. *J. Virol.* 75, 3095-3104 (2001).

37. Zahran,M.A., Kovacs,L., el,S., I, Pedersen,E.B. & Nielsen,C. The potential of aspirin in prodrug synthesis: a new potential delivery system of AZT and FLT. *Arch. Pharm. (Weinheim)* 329, 417-420 (1996).

38. St Clair,M.H. *et al.* 3'-Azido-3'-deoxythymidine triphosphate as an inhibitor and substrate of purified human immunodeficiency virus reverse transcriptase. *Antimicrob. Agents Chemother.* 31, 1972-1977 (1987).

39. Kaul,M., Garden,G.A. & Lipton,S.A. Pathways to neuronal injury and apoptosis in HIV-associated dementia. Nature 410, 988-994 (2001).

Example 2 APHS can act synergistically with clinically available HIV-1 reverse transcriptase and protease inhibitors and is active against several drug-resistant HIV-1 *strains in vitro*

Several steps in human immunodeficiency virus (HIV) replication are susceptible to the action of anti-HIV agents. Current anti-HIV therapy uses two classes of drugs: reverse transcriptase (RT) inhibitors (RTI) and protease inhibitors (PI) [1,2]. The first class of drugs comprises two subgroups: (i) nucleoside/nuceoside RTI (NRTI) such as zidovudine (AZT), lamivudine (3TC), didanosine (ddI), stavudine (d4T), zalcitabine (ddC), abacavir and tenofovir disoproxil fumarate and (ii) non-nucleoside RT inhibitors (NNRTI) such as efavirenz (EFV), delavirdine and nevirapine. Examples of PI are indinavir (IDV), ritonavir (RTV), saquinavir, amprenavir, nelfinavir and lopinavir. NRTI prevent elongation of the DNA chain, which results in DNA chain termination. NNRTI bind to an allosteric site of reverse transcriptase adjacent to the polymerase active site, rendering it inactive. Protease inhibitors bind to the viral protease and in this way they prevent processing of virus proteins and virus maturation [3]. Although use of these drugs rapidly reduces HIV RNA levels, long-term treatment leads to the emergence of drug resistance and toxicity [4-10]. HIV reverse transcriptase lacks proofreading activity, which results in a high mutation rate of $4 \times 10^{-5}$ mutations per bp [11]. Since the estimated average of HIV-1 production is $10.5\times 10^{9}$ virions per day [12], replication competent drug-resistant viruses are rapidly selected.
Current anti-HIV therapy consists of combinations of three or more antiretroviral drugs, the so called highly active antiretroviral therapy (HAART). This therapy is more efficient in suppressing viral production than single drug therapy [13-17]. Also, synergistic interaction, defined as an combined effect greater than expected from the additive effect from the individual drugs [18], has been shown for different combinations of anti-HIV drugs [19-23]. However, drug combination can also have undesirable side-effects such as emergence of cross-resistance mutations, combined toxicity and, as a consequence, poor adherence to the treatment and frequent alteration in the panel of agents used [10,24-29]. Intervention at more than a single step in the HIV replication cycle will probably be more efficient in suppressing viral replication and avoiding resistance. Therefore, in the last few years several anti-oxidative compounds, anti-proliferative compounds, anti-inflammatory compounds and compounds that target new viral and cellular factors involved in virus replication were proposed as anti-HIV agents [1,3,4,30-35]. Besides a good antiviral activity, new drugs should also be pharmacologically compatible with other anti-HIV drugs, show minimal toxicity and inhibit HIV strains that are resistant to clinically available drugs [3].

We have previously found that o-(acetoxyphenyl)hept-2-ynyl sulfide (APHS) (Figure 2.1), a selective nonsteroidal anti-inflammatory drug (NSAID) [36], can inhibit the replication of several HIV-1 strains *(Ba-L, HXB2* and *AT)* in primary cells (peripheral blood mononuclear cells, monocyte-derived macrophages and peripheral blood lymphocytes) *in vitro* by interfering with the reverse transcription process [37]. APHS 50% inhibitory concentration ($IC_{50}$) for HIV-1 was 6 μM, while its 50% toxic concentration for primary cells was 105 μM.

The aim of this study was to investigate whether APHS shows synergistic interactions with the clinically available drugs AZT, 3TC, EFV, IDV and RTV and whether APHS can inhibit the replication of NRTI and PI-resistant HIV-1strains.

Materials and methods

*Isolation of primary cells*

Donor peripheral blood mononuclear cells (PBMC) were isolated from heparinized blood from HIV-1-, HIV-2-, and hepatitis B-seronegative donors and obtained on Ficoll-Isopaque density gradients. To prepare a PBMC mixed batch, PBMC isolated from six donors were pooled together in RPMI 1640 medium (Gibco, Invitrogen, Paisley, Scotland) supplemented with 10% dimethyl sulfoxide (DMSO; Merck, Darmstadt, Germany), 20% fetal calf serum (FCS; Invitrogen) and 10 μg/ml gentamycin (Invitrogen) and frozen at -140 °C. Cells were thawed and cultured for 4 days prior to the experiment in RPMI 1640 medium supplemented with 10% FCS, and 10 μg/ml gentamycin containing 2 μg/ml lectin from *Phaseolus vulgaris* (PHA, Sigma Chemie, Zwijndrecht, The Netherlands) at 37 °C and 5% $CO_2$.

*Compounds*

APHS was supplied by Dr. L.J. Marnett. Synthesis details are described by Kalgutkar et al.[38]. APHS was

diluted in aliquots in 100% ethanol, topped with argon gas and stored at - 20 °C. The concentration of ethanol during incubations never exceeded 0.1%. At this concentration, ethanol did not affect HIV-1 replication or cellular viability (data not shown). AZT (Sigma), IDV (Merck) and RTV (Abbott Laboratories S.A., Baar, Switzerland) were diluted in DMSO and 3TC (Glaxo Welcome, Research Triangle Park, NC. USA) and EFV (Merck) were diluted in water. The concentration of DMSO during incubations never exceeded 0.001% and no effect on HIV-1 replication or cellular viability was observed at this concentration (data not shown).

*HIV-1 infection of PBMC*

PHA-stimulated PBMC were washed twice to remove PHA and incubated for 7 days in a concentration of $5 \times 10^5$ cells/ml with HIV-$1_{Ba\text{-}L}$ at a multiplicity of infection (M.O.I.) of 0.0025, in the presence of APHS and/or other drugs and 10 U/ml recombinant IL-2 (Roche Diagnostics GmbH, Mannheim, Germany), at 37°C and 5% $CO_2$. To correct for the input virus, an extra control consisting of medium containing the same amount of input virus as added to the cells was included in the experiment. The amount of p24 in this control was subsequently subtracted from the p24 values of the samples in order to obtain the exact amount of p24 produced by the cells.

*p24-core antigen quantification by ELISA*

After 7 days incubation, samples of the supernatants were collected, inactivated by addition of Empigen (Calbiochem, La Jolla, CA, USA) and by heat inactivation at 56°C for 30 min. p24-core antigen concentration was determined by an ELISA (AMPAK™, DAKO, Cambridgeshire, UK) as described previously [39,40]. The optical density values were converted into p24 concentration (ng/ml) with the use of a calibration curve made by serial dilutions of recombinant p24 protein (NIBSC, UK) that was submitted to the same treatment as the samples.

*Determination of viability of PBMC*

After 7 days incubation, the metabolic activity of PBMC from the HIV-1 infection model was assessed by a cellular viability assay as described previously [41]. Shortly, 150 µg/ml tetrazollium salt 3-(4,5-dimethylthiazol-2-yl)-2,5-diphenyltetrazolliumbromide (MTT) (Sigma) is added to the cells. During the subsequent 2h incubation period metabolic active cells convert MTT into blue formazan crystals. Afterwards, ¾ of the supernatant was gently removed and substituted by stop buffer consisting of 90% 2-propanol, 10% triton X-100 and 0.4% HCl (Merck, Darmstadt, Germany). When all formazan crystals were dissolved optical density was measured at 550 nm and using 630 nm as reference.

*Determination of $IC_{50}$ of the compounds*

The ability of each compound to decrease p24 antigen was expressed as $IC_{50}$. $IC_{50}$ was calculated using the computer software program CalcuSyn for Windows (Biosoft, Cambridge, UK) according to the method of Chou and Talalay [18,42]. This program uses the median-effect equation to produce dose-effect curves:

$$f_a = 1/[1 + (D_m/D)^m] \quad \text{[equation 1]}$$

where $f_a$ represents fraction affected by the dose (reduction of p24 at a certain drug concentration expressed in decimals), $D_m$ is the median effect dose (same as $IC_{50}$), $D$ is the dose of the drug and m is the sigmoidicity coefficient of the dose-effect curve. Data was accepted when the linear correlation coefficient of the median-effect plot based on experimental data was > 0.90.

*Combination of HIV-1 inhibitors*

In the combination experiments, PBMC were infected as described above and seeded in the presence of multiple-diluted fixed ratios of a combination of APHS and the clinically available drugs AZT, 3TC, EFV, IDV and RTV, in duplicate wells, according to the checker board design (Table 2.1). Before the experiments were performed, $IC_{50}$ of all drugs were determined as described before in order to choose the appropriate dosage ranges for the combinations with APHS. Concentrations of APHS, AZT, 3TC, EFV, IDV and RTV ranged between 0.75 and 24 microM, 0.63 and 10 nM, 0.63 and 10 nM, 37.5 and 600 pM, 0.63 and 10 nM and 0.88 and 14 nM, respectively. After 7 days of incubation, p24 antigen in culture supernatant was quantified by ELISA and cellular viability was assessed by a cytotoxicity assay.

$f_a$ was calculated for each drug combination after p24 quantification. Using the CalcuSyn program, dose-effect curves were plotted for each drug combination, i.e., each dose was plotted against the corresponding $f_a$ value. Using the multiple drug-effect equation, which is based on the median-effect equation and the isobologram method, $IC_{50}$ was calculated for both drugs alone and in combination at their equipotent ratio ($IC_{50}$ APHS: $IC_{50}$ drug). Data was accepted when the linear correlation coefficient of the median-effect plot based on experimental data was > 0.90. The combination index (CI), a quantitative measure of drug interaction, was calculated for each $f_a$ at three different drug ratios (1:2, 1: 1 and 2 : 1) based on $IC_{50}$ values of each drug used alone (e.g., 1 : 1 drug ratio represents the equipotent ratio ($IC_{50}$APHS : $IC_{50}$ drug)), according to the following equation:

$$CI_x = (D)_1 / (D_x)_1 + (D)_2 / (D_x)_2 + (D)_1 (D)_2 / (D_x)_1 (D_x)_2 \quad \text{[equation 2]}$$

$CI_X$ stands for the combination index at x% inhibition, $(D)_1$ and $(D)_2$ are the doses from drug 1 and 2 used when inhibiting x% in combination and $(D_x)_1$ and $(D_x)_2$ are the doses of drug 1 and 2 alone, inhibiting x% (calculated from equation 1). A drug combination is additive when CI≈1, synergistic when CI<1 (combined effect is greater than the additive effect) and antagonistic is indicated when CI>1 (combined effect is less than the additive effect). As the range of CI << 1, the degree of synergistic interaction gets stronger [18]. CI values were calculated using the mutually nonexclusive assumption.

*Drug susceptibility assay*

MT-2 cells (Medical Research Counsil, London, UK) were cultured in RPMI 1640 medium supplemented with 10% FCS and 10 microg/ml gentamycin and passaged once a week until a maximum of 20 passages. The cells were maintained at 37°C, 5% $CO_2$. One day prior to the experiments, the cells were passaged 1:1 in a concentration of $1 \times 10^6$ cells/ml.

*HXB2* is the molecular clone of the first laboratory HIV-1 isolate. The genes that contain the drug resistance mutations are excised from the clinical isolates and cloned into the genetic background of *HXB2.* Thus all tested HIV-1 strains have the same genetic background. *41 + 215Y* is an AZT-resistant HIV-1 strain as described by Jeeninga and colleagues [43]. This strain contains one amino-acid (a.a.) change at codon 41 (Met is substituted by Leu) and one a.a. change at codon 215 (Thr is substituted by Tyr). *184V* is a 3TC-resistant HIV-1 strain as described by Schuurman and colleagues [5]. This strain contains one a.a. change at codon 184 (Met is substituted by Val). Strain *3096* is a RTI resistant HIV-1 strain as described by de Jong and colleagues [27]. This strain contains an insertion of two a.a. between codons 68 and 69 of RT as well as an a.a. change at codon 67. Phenotypic resistance analysis showed high levels of resistance to AZT, 3TC and d4T and moderate levels of resistance to ddI and ddC. Strain *4602* is a RTV-resistant HIV-1 strain as described by Nijhuis and colleagues [9]. It contains the following mutations: 36I, 54V, 71V, and 82T (4).

The susceptibility of these HIV-1 strains for APHS was analyzed in a cell-killing assay (MTT assay) as described previously [44]. Briefly, MT-2 cells in a concentration of $0.4 \times 10^6$ cells/ml were seeded with or without HIV-1 wild-type or drug-resistant mutants at a MOI of 0.002 or 0.006 and in the presence of increasing concentrations of APHS and maintained at 37 °C, 5% $CO_2$. After 5 days of incubation a cellular viability assay was performed as described above. In this case, the amount of formazan reflects the number of cells protected by the drug against killing by the virus and is used as a read out for drug susceptibility. The $IC_{50}$ of APHS for the wild-type and for each of the virus mutants was determined using a computer software program.

Results

*Combination of HIV-1 inhibitors*

Antiviral activity of APHS in combination with clinically available drugs. Antiviral activity of APHS in combination with the RTI AZT, 3TC and EFV, and the PI IDV and RTV, was studied. PBMC, infected with HIV-$1_{Ba\text{-}L}$, were incubated with increasing concentrations of APHS and drugs and diluted according to the checker board design (Table 2.1). After 7 days, viral production and cellular viability were quantified. All fixed ratio combinations of drugs showed no cytotoxicity as assessed by a cytotoxicity assay (data not shown). $IC_{50}$ for individual drugs and their combination were determined (Table 2.2). The $IC_{50}$ of alone varied from 10 to 15 μM (mean 13 μM). When used alone, the $IC_{50}$ of AZT, 3TC, EFV, IDV and RTV were 3 nM, 10 nM, 1 nM, 11 nM and 6 nM, respectively. $IC_{50S}$ of these clinically available drugs were comparable to those previously described in literature [45]. The $IC_{50}$ of APHS dropped to 4, 5 and 6 μM (mean 5 μM) when used in combination with another drug. The $IC_{50}$ of AZT, 3TC, EFV, IDV and RTV dropped to 2, 3, 0.2, 5 and 3 nM, respectively. Although a definite trend was evident the only statistically significant decreases in $IC_{50S}$

were observed for IDV when used in combination with APHS and for APHS when used in combination with EFV ($p<0.05$ according to Student's *t*-test).
In order to investigate possible synergistic interactions between APHS and RTI or PI, combination indices (CI) were determined for each combination of drugs at three different drug ratios. These drug ratios were based on the $IC_{50}$ of the individual drugs. Additive interaction between multiple drugs is indicated when CI is approximately 1, synergistic interaction is indicated when CI < 1 and antagonistic interaction is indicated when CI > 1. For each combination, CI values for three drug ratios were determined at the calculated 50, 75 and 95% of HIV-1 inhibition levels. Results are shown in Table 2.3. The combination of APHS with AZT yielded moderate synergistic to nearly additive interactions at the 1 : 2 drug ratio. At the 1 : 1 drug ratio, moderate synergistic to synergistic interactions were observed. Synergistic interactions were found at the 50 and 75% of inhibition at the 2 : 1 drug ratio. For 95% of inhibition no conclusions could be drawn due to the high sample variation. Thus, the combined effect of APHS with AZT was additive to synergistic. The combination of APHS and 3TC at the 1 : 2 drug ratio showed slight synergism interactions for 50% of inhibition but strong synergism interactions for 75 and 95% of inhibition. At the 1 : 1 drug ratio, moderate synergistic interactions were observed for 50% of inhibition but strong synergistic interactions were observed for 75 and 95% of inhibition. The 2 : 1 drug ratio showed slight antagonism interactions at 50% of inhibition, but synergistic to strong synergistic interactions were found at higher effect levels. So, the combination of APHS and 3TC yielded synergistic to strong synergistic interactions at higher levels of inhibition in all drug ratios tested. The combination of APHS and EFV showed nearly additive interactions for 50 and 75% of inhibition but synergistic interactions were observed for 95% of inhibition at the 1 : 2 drug ratio. At the 1 : 1 drug ratio, synergistic interactions for 50 and 75% of inhibition were observed but strong synergistic interactions were observed for 95% of inhibition. At the 2 : 1 drug ratio, sample variation was too high for conclusions to be drawn. Thus, the combination APHS-EFV showed synergistic interactions at higher levels of inhibition for the 1 : 2 and 1 : 1 drug ratios. The combination of APHS and IDV yielded moderate synergistic interactions at the 1 : 2 drug ratio. At the 1 : 1 drug ratio, the combination showed synergistic to nearly additive interactions at the lowest percentage of inhibition levels. At 95% of inhibition no conclusions could be drawn due to sample variation. At the 2 : 1 drug ratio, slight synergistic interactions were observed for 50% of inhibition but synergistic interactions were observed for 75 and 95% of inhibition. In conclusion, the combination of APHS and IDV showed synergistic to additive interactions for all drug ratios tested. For the combination of APHS and RTV at the 1 : 2 drug ratio, slight synergistic interactions were observed for 50% of inhibition, synergistic interactions were observed for 75% of inhibition and slight antagonistic interactions were observed for 95% of inhibition. At the 1 : 1 drug ratio, moderate antagonistic interactions were observed for 50% of inhibition, nearly additive interactions were observed for 75% of inhibition and slight synergistic were found for 95% of inhibition. At the 2 : 1 drug ratio slight synergistic interactions were found for 75% of inhibition and synergistic interactions were observed for 95% of inhibition. At 50% of inhibition no conclusions could be drawn due to sample variation. Thus, the combination of APHS and RTV resulted in synergistic to moderate antagonistic interactions.

*Drug susceptibility assay*

To determine the susceptibility of wild-type HIV-1 and several NRTI or PI-resistant HIV- strains for APHS, a drug susceptibility assay was performed. This assay gives information not only about antiviral activity of APHS but also about its cytotoxicity patterns. The $IC_{50}$ of APHS for wild-type HIV-1 and drug-resistant strains are depicted in Table 2.4. The $IC_{50}$ of APHS for the wild type strain *HXB2* was 10μM. The $IC_{50}$ of APHS for the AZT-resistant strain *41+215Ywas* 2 μM. The $IC_{50}$ of APHS for the 3TC-resistant strain *184V* was 5 μM. The $IC_{50}$ of APHS for the AZT, 3TC, d4T, ddI and ddC-resistant strain *3096* was 4 μM. The $IC_{50}$ of APHS for the RTV-resistant strain *4602* was 5 μM. APHS did not show any significant toxicity for MT2 cells at the tested concentrations and therefore it can be concluded that APHS was capable of inhibiting the replication of all four HIV-1 strains tested.

We have shown that APHS, a novel selective NSAID derived from aspirin, can inhibit HIV-1 replication *in vitro* by interfering with the reverse transcription process [37]. It is important to investigate the interaction between new drugs and currently used drugs, especially the determination of the possible synergistic interactions and the possible adverse effects arising from the combination. In this study there was a clear trend towards decrease in $IC_{50}$ of the drugs when used in combination then when used alone. This means that the same effect can be obtained with lower doses of the individual drugs and, in this way, toxic side-effects are also minimized.
There are several methods described in literature to determine synergistic interaction [46]. We used the computer program CalcuSyn [42], which is based on the median-effect principle as described before [18]. This program is simple to use, it is not limited by the number, effect and interactions of the drugs tested and it only requires a minimum of data points to analyze drug interaction. CalcuSyn is often used in combination studies of anti-HIV agents [20,45,47-49]. It has been shown that the results obtained by this method are comparable to that of other methods such as MacSynergy, especially if more than one drug ratio was employed [20,48].

Although combinations of APHS with the RTI AZT, 3TC and EFV showed for all drug ratios slight antagonistic

to synergistic interactions at the 50% inhibition level, moderate synergistic to strong synergistic interactions were observed at the 95% inhibition level. The fact that synergistic interactions between APHS and RTI become stronger at higher percentages of inhibition is important for in vivo therapeutics when a higher percentage of inhibition is desired. The strongest synergistic interactions were found between APHS and 3TC. Moderate antagonistic to synergistic interactions were found between APHS and the PI IDV and RTV. No correlation was found between the percentage of inhibition level and the degree of interaction between APHS and PI. Slightly different effects were observed at different drug ratios for all combinations tested. This reflects the importance of choosing the most effective drug ratio for a combination of antiviral drugs.

The strong synergistic interactions between APHS and RTI are probably due to the fact that APHS also inhibits the reverse transcription process. There are many studies that report a benefit arising from the combination of NRTI and NNRTI [19,50-54]. Although the mechanism for synergy seen by the use of NNRTI and NRTI is not known, it is conceivable that the interaction of the NNRTI with the RT at the NNRTI binding site, which has been shown to cause conformational distortion of the catalytic aspartate triad [55], may allow improved incorporation of the NRTI into the growing DNA molecule, leading to more efficient chain termination or decreased rates of phosphorolytic removal of the terminator. APHS can also interact synergistically with PI. In acutely-infected cell cultures, individual cells are present at different stages of the HIV-1 life cycle. Several other RTI have shown synergistic interactions with PI [20,23,49,50,56,57]. Combination of antiviral compounds with different targets can inhibit HIV-1 replication in a greater proportion of cells at different stages of viral replication resulting in an increased viral suppression [56,20,23]. Importantly, no strong antagonism was found between APHS and RTI or PI. Although *in vivo* studies will have to be conducted, these data suggest that APHS can safely be used in combination with RTI and PI.

New drugs should not only be pharmacologically compatible with currently available drugs but also be able to inhibit the replication of drug-resistant HIV strains. Some compounds with this characteristic are being developed [45,48,58-60]. In this study APHS could inhibit the replication of both wild-type and several NRTI and PI-resistant HIV-1 strains. Since the mutations tested are very common *in vivo,* APHS may prove to be very useful against HIV-1 strains that acquired resistance to clinically available anti-HIV-1 drugs.

Since APHS is a derivative of aspirin and since previous studies in a rat air pouch model indicated that APHS concentrations up to 100mg/kg are not toxic [36], it is reasonable to believe that it will be safe to use APHS *in vivo.* In conclusion, since previous studies have shown that APHS has minimal toxicity *in-vivo* and a good anti-HIV activity and since this study showed that APHS acts synergistically with clinically available RTI and PI and is able to inhibit both wild-type and RTI and PI-resistant HIV-1 strains, APHS is a very promising candidate for anti-HIV therapy *in vivo.*

Table 2.1. Experimental checker board design for two drug combinations. Three different drug ratios are shown (1 : 2, 1 : 1, 2 : 1).

→ APHS

↓ Drug

| | 0 | $0.125 \times IC_{50}$ | $0.25 \times IC_{50}$ | $0.5 \times IC_{50}$ | $1 \times IC_{50}$ | $2 \times IC_{50}$ |
|---|---|---|---|---|---|---|
| 0 | | | | | | |
| $0.125 \times IC_{50}$ | | 1 : 1 | 2 : 1 | | | |
| $0.25 \times IC_{50}$ | | 1 : 2 | 1 : 1 | 2 : 1 | | |
| $0.5 \times IC_{50}$ | | | 1 : 2 | 1 : 1 | 2 : 1 | |
| $1 \times IC_{50}$ | | | | 1 : 2 | 1 : 1 | 2 : 1 |
| $2 \times IC_{50}$ | | | | | 1 : 2 | 1 : 1 |

Table 2.2.

| $IC_{50}$ of and clinically available anti-HIV drugs alone and after combination at their equipotent ratio. | | | | |
|---|---|---|---|---|
| | $IC_{50}$[a] | | | |
| | Alone | Combination[b] | | |
| | APHS (μM) | Drug (nM) | APHS (μM) | Drug (nM) |
| APHS/AZT | 15 ±7[c] | 3±1 | 6±2 | 2±0.4 |
| APHS/3TC | 10±4 | 10±3 | 4±1 | 3±1 |
| APHS/EFV | 14±3 | 1±0.3 | 5±1* | 0.2±0.1 |
| APHS/IDV | 12±4 | 11±1 | 4±1 | 5±1* |
| APHS/RTV | 15 ±3 | 6±2 | 6±1 | 3±1 |

[a] $IC_{50}$ represents the 50% inhibiting concentration.
[b] Combinations were performed at the equipotent ratio (1 : 1 or $IC_{50}$APHS : $IC_{50}$ drug).
[c] Values represent mean ± SEM of at least three independent experiments.
* *p*<0.05 (according to Student's *t*-test).

Table 2.3.

| CI values for two-drug combinations of APHS with RTI AZT, 3TC and EFV and with the PI IDV and RTV. | | | | |
|---|---|---|---|---|
| Combination | Drug ratio [a] | CI at the following levels of HIV-1 inhibition (%)[b] | | |
| | | 50 | 75 | 95 |
| APHS/AZT | 1 : 2 | 1.0±0.2[c] | 1.1±0.3 | 0.8±0.2 |
| | 1 : 1 | 0.8±0.02 | 0.5±0.06 | 0.5±0.1 |
| | 2 : 1 | 0.5±0.1 | 0.6±0.2 | N.D[d] |
| APHS/3TC | 1 : 2 | 0.9±0.3 | 0.3 ±0.1 | 0.2±0.1 |
| | 1 : 1 | 0.8 ±0.1 | 0.4±0.1 | 0.2±0.1 |
| | 2 : 1 | 1.2±0.3 | 0.5±0.3 | 0.3±0.3 |
| APHS/EFV | 1 : 2 | 1.1±0.2 | 1.1 ± 0.4 | 0.7±0.2 |
| | 1 : 1 | 0.7 ±0.1 | 0.6±0.1 | 0.3 ±0.04 |
| | 2 : 1 | N.D. | N.D. | N.D. |
| APHS/IDV | 1 : 2 | 0.8±0.2 | 0.7 ±0.2 | 0.8 ±0.5 |
| | 1 : 1 | 0.6 ±0.04 | 1.0 ±0.3 | N.D. |
| | 2 : 1 | 0.9 ±0.2 | 0.6 ±0.1 | 0.7±0.1 |
| APHS/RTV | 1 : 2 | 0.9 ±0.5 | 0.7 ±0.02 | 1.3 ±0.5 |
| | 1 : 1 | 1.3 ±0.5 | 1.0 ±0.4 | 0.9 ±0.4 |
| | 2 : 1 | N.D. | 0.9 ±0.1 | 0.7 ±0.2 |

[a] Drug ratio are based on $IC_{50}$ values of each drug used alone. 1:1 drug ratio represents the equipotent ratio ($IC_{50}$APHS : $IC_{50}$ drug).
[b] CI's were calculated according to the method described by Chou and Talalay: <0.1 (very strong synergism), 0.1-0.3 (strong synergism), 0.3-0.7 (synergism), 0.7-0.85 (moderate synergism), 0.85-0.9 (slight synergism), 0.9-1.1 (nearly additive), 1.1-1.2 (slight antagonism), 1.2-1.45 (moderate antagonism), 1.45-3.3 (antagonism), 3.3-10 (strong antagonism)and >10 (very strong antagonism).
[c] Values represent means ± SEM for at least three independent experiments.
[d] CI values could not be determined.

Table 2.4.

| Susceptibility of an HIV-1 wild-type *(HXB2)* and of different HIV-1 reverse transcriptase (AZT 3TC, d4T, ddI and ddC) and protease (RTV) resistant virus for APHS. | | |
|---|---|---|
| Virus | Resistance | $IC_{50}$ (μM)[a] |
| *HXB2* | | 10 ± 2.2[b] |
| *41+215Y* | AZT | 2.1 ± 1.0 |
| *184V* | 3TC | 5 ± 1.3 |
| *3096* | (high to) AZT, 3TC and d4T (moderate to) ddI and ddC | 4 ± 1.8 |
| *4602* | RTV | 5 ± 1.1 |

[a] $IC_{50}$ represents the 50% inhibiting concentration.
[b] Values represent means ± SEM for at least three independent experiments.

References with example 2

1. De Clercq,E. Novel compounds in preclinical/early clinical development for the treatment of HIV infections. *Rev. Med. Virol.* 10, 255-277 (2000).

2. Tavel,J.A., Miller,K.D. & Masur,H. Guide to major clinical trials of antiretroviral therapy in human immunodeficiency virus-infected patients: protease inhibitors, non-nucleoside reverse transcriptase inhibitors, and nucleotide reverse transcriptase inhibitors. *Clin. Infect. Dis.* 28, 643-676 (1999).

3. Richman,D.D. HIV chemotherapy. Nature 410, 995-1001 (2001).

4. Baba,M. Cellular factors as alternative targets for inhibition of HIV-1. *Antiviral Res.* 33, 141-152 (1997).

5. Schuurman,R. *et al.* Rapid changes in human immunodeficiency virus type 1 RNA load and appearance of drug-resistant virus populations in persons treated with lamivudine (3TC). *J. Infect. Dis.* 171, 1411-1419 (1995).

6. Maxeiner,H.G. *et al.* Selection of Zidovudine resistance mutations and escape of human immunodeficiency virus type 1 from antiretroviral pressure in Stavudine-treated pediatric patients. *J. Infect. Dis.* 185, 1070-1076 (2002).

7. Carr,A. *et al.* A syndrome of peripheral lipodystrophy, hyperlipidaemia and insulin resistance in patients receiving HIV protease inhibitors. *AIDS* 12, F51-F58 (1998).

8. Huang,H., Chopra,R., Verdine,G.L. & Harrison,S.C. Structure of a covalently trapped catalytic complex of HIV-1 reverse transcriptase: implications for drug resistance. *Science* 282, 1669-1675 (1998).

9. Nijhuis,M. *et al.* Increased fitness of drug resistant HIV-1 protease as a result of acquisition of compensatory mutations during suboptimal therapy. *AIDS* 13, 2349-2359 (1999).

10. Notermans,D.W., van Leeuwen,R. & Lange,J.M. Treatment of HIV infection. Tolerability of commonly used antiretroviral agents. *Drug Saf* 15, 176-187 (1996).

11. Mansky,L.M. The mutation rate of human immunodeficiency virus type 1 is influenced by the vpr gene. *Virology* 222, 391-400 (1996).

12. Perelson,A.S., Neumann,A.U., Markowitz,M., Leonard,J.M. & Ho,D.D. HIV-1 dynamics in vivo: virion clearance rate, infected cell life-span, and viral generation time. *Science* 271, 1582-1586 (1996).

13. Lipsky,J.J. Antiretroviral drugs for AIDS. *Lancet* 348, 800-803 (1996).

14. Hammer,S.M. *et al.* A controlled trial of two nucleoside analogues plus indinavir in persons with human immunodeficiency virus infection and CD4 cell counts of 200 per cubic millimeter or less. AIDS Clinical Trials Group 320

Study Team. *N. Engl. J. Med.* 337, 725-733 (1997).

15. Mocroft,A. *et al.* Changing patterns of mortality across Europe in patients infected with HIV-1. EuroSIDA Study Group. *Lancet* 352, 1725-1730 (1998).

16. Palella,F.J., Jr. *et al.* Declining morbidity and mortality among patients with advanced human immunodeficiency virus infection. HIV Outpatient Study Investigators. *N. Engl. J. Med.* 338, 853-860 (1998).

17. Henry,K. *et al.* A randomized, controlled, double-blind study comparing the survival benefit of four different reverse transcriptase inhibitor therapies (three-drug, two-drug, and alternating drug) for the treatment of advanced AIDS. AIDS Clinical Trial Group 193A Study Team. J. *Acquir. Immune. Defic. Syndr. Hum. Retrovirol.* 19, 339-349 (1998).

18. Chou,T.C. & Talalay,P. Quantitative analysis of dose-effect relationships: the combined effects of multiple drugs or enzyme inhibitors. *Adv. Enzyme Regul.* 22, 27-55 (1984).

19. Maga,G. *et al.* Potentiation of inhibition of wild-type and mutant human immunodeficiency virus type 1 reverse transcriptases by combinations of nonnucleoside inhibitors and d- and L-(beta)-dideoxynucleoside triphosphate analogs. *Antimicrob. Agents Chemother.* 45, 1192-1200 (2001).

20. Deminie,C.A. *et al.* Evaluation of reverse transcriptase and protease inhibitors in two-drug combinations against human immunodeficiency virus replication. *Antimicrob. Agents Chemother.* 40, 1346-1351 (1996).

21. Villahermosa,M.L., Martinez-Irujo,J.J., Cabodevilla,F. & Santiago,E. Synergistic inhibition of HIV-1 reverse transcriptase by combinations of chain-terminating nucleotides. *Biochemistry* 36, 13223-13231 (1997).

22. Tremblay,C., Merrill,D.P., Chou,T.C. & Hirsch,M.S. Interactions among combinations of two and three protease inhibitors against drug-susceptible and drug-resistant HIV-1 isolates. *J. Acquir. Immune. Defic. Syndr.* 22, 430-436 (1999).

23. Snyder,S., D'Argenio,D.Z., Weislow,O., Bilello,J.A. & Drusano,G.L. The triple combination indinavir-zidovudine-lamivudine is highly synergistic. *Antimicrob. Agents Chemother.* 44, 1051-1058 (2000).

24. Carr,A., Miller,J., Law,M. & Cooper,D.A. A syndrome of lipoatrophy, lactic acidaemia and liver dysfunction associated with HIV nucleoside analogue therapy: contribution to protease inhibitor-related lipodystrophy syndrome. *AIDS* 14, F25-F32 (2000).

25. Max,B. & Sherer,R. Management of the adverse effects of antiretroviral therapy and medication adherence. *Clin. Infect Dis.* 30 Suppl 2, S96-116 (2000).

26. Monno,L., Appice,A., Cavaliere,R., Scarabaggio,T. & Angarano,G. Highly active antiretroviral therapy failure and protease and reverse transcriptase human immunodeficiency virus type 1 gene mutations. *J. Infect Dis.* 180, 568-571 (1999).

27. de Jong,J.J. *et al.* Insertion of two amino acids combined with changes in reverse transcriptase containing tyrosine-215 of HIV-1 resistant to multiple nucleoside analogs. *AIDS* 13, 75-80 (1999).

28. Rousseau,M.N. *et al.* Patterns of resistance mutations to antiretroviral drugs in extensively treated HIV-1-infected patients with failure of highly active antiretroviral therapy. *J. Acquir. Immune. Defic. Syndr.* 26, 36-43 (2001).

29. Maga,G. & Spadari,S. Combinations against combinations: associations of anti-HIV 1 reverse transcriptase drugs challenged by constellations of drug resistance mutations. *Curr. Drug Metab* 3, 73-95 (2002).

30. Daelemans,D., Vandamme,A.M. & De Clercq,E. Human immunodeficiency virus gene regulation as a target for antiviral chemotherapy. *Antivir. Chem. Chemother.* 10, 1-14 (1999).

31. Lori,F. *et al.* Immune restoration by combination of a cytostatic drug (hydroxyurea) and anti-HIV drugs (didanosine and indinavir). *AIDS Res. Hum. Retroviruses* 15, 619-624 (1999).

32. Martin,M. *et al.* PMS-601, a new platelet-activating factor receptor antagonist that inhibits human immunodeficiency virus replication and potentiates zidovudine activity in macrophages. *Antimicrob. Agents Chemother.* 44, 3150-3154 (2000).

33. Rozera,C. *et al.* Inhibition of HIV-1 replication by cyclopentenone prostaglandins in acutely infected human cells. Evidence for a transcriptional block. *J. Clin. Invest* 97, 1795-1803 (1996).

34. Bourinbaiar,A.S. & Lee-Huang,S. The non-steroidal anti-inflammatory drug, indomethacin, as an inhibitor of HIV replication. *FEBS Lett.* 360, 85-88 (1995).

35. Macilwain,C. Aspirin on trial as HIV treatment. Nature 364, 369 (1993).

36. Kalgutkar,A.S. *et al.* Aspirin-like molecules that covalently inactivate cyclooxygenase-2. *Science* 280, 1268-1270 (1998).

37. Pereira,C.F. *et al.* Aspirin-like molecules that inhibit human immunodeficiency virus-1 replication. *Antivir. Res., in press* (2003).

38. Kalgutkar,A.S., Kozak,K.R., Crews,B.C., Hochgesang,G.P.J. & Marnett,L.J. Covalent modification of cyclooxygenase-2 (COX-2) by 2-acetoxyphenyl alkyl sulfides, a new class of selective COX-2 inactivators. *J. Med. Chem.* 41, 4800-4818 (1998).

39. McKeating,J.A., McKnight,A. & Moore,J.P. Differential loss of envelope glycoprotein gp 120 from virions of human immunodeficiency virus type 1 isolates: effects on infectivity and neutralization. *J. Virol.* 65, 852-860 (1991).

40. Moore,J.P., McKeating,J.A., Weiss,R.A. & Sattentau,Q.J. Dissociation of gp120 from HIV-1 virions induced by soluble CD4. *Science* 250, 1139-1142 (1990).

41. Pauwels,R. *et al.* Rapid and automated tetrazolium-based colorimetric assay for the detection of anti-HIV compounds. *J. Virol. Methods* 20, 309-321 (1988).

42. Chou,T.C. & Hayball,M.P. (BIOSOFT, Cambridge,UK,1996).

43. Jeeninga,R.E., Keulen,W., Boucher,C., Sanders,R.W. & Berkhout,B. Evolution of AZT resistance in HIV-1: the 41-70 intermediate that is not observed in vivo has a replication defect. *Virology* 283, 294-305 (2001).

44. Boucher,C.A. *et al.* Human immunodeficiency virus type 1 drug susceptibility determination by using recombinant viruses generated from patient sera tested in a cell-killing assay. *Antimicrob. Agents Chemother.* 40, 2404-2409 (1996).

45. Essey,R.J., McDougall,B.R. & Robinson,W.E., Jr. Mismatched double-stranded RNA (polyI-polyC(12)U) is synergistic with multiple anti-HIV drugs and is active against drug-sensitive and drug-resistant HIV-1 in vitro. *Antiviral Res.* 51, 189-202 (2001).

46. Greco,W.R., Bravo,G. & Parsons,J.C. The search for synergy: a critical review from a response surface perspective. *Pharmacol. Rev.* 47, 331-385 (1995).

47. Georgiou,N.A. *et al.* The chemotherapeutic agent bleomycin in a two-drug combination with zidovudine, ritonavir or indinavir synergistically inhibits HIV Type-1 replication in peripheral blood lymphocytes. *Int. J. Antimicrob. Agents* 18, 513-518 (2001).

48. Chong,K.T. & Pagano,P.J. In vitro combination of PNU-140690, a human immunodeficiency virus type 1 protease inhibitor, with ritonavir against ritonavir-sensitive and -resistant clinical isolates. *Antimicrob. Agents Chemother.* 41, 2367-2373 (1997).

49. Lamarre,D. *et al.* Antiviral properties of palinavir, a potent inhibitor of the human immunodeficiency virus type 1 protease. *Antimicrob. Agents Chemother.* 41, 965-971 (1997).

50. Buckheit,R.W., Jr., Russell,J.D., Xu,Z.Q. & Flavin,M. Anti-HIV-1 activity of calanolides used in combination with other mechanistically diverse inhibitors of HIV-1 replication. *Antivir. Chem. Chemother.* 11, 321-327 (2000).

51. King,R.W., Klabe,R.M., Reid,C.D. & Erickson-Viitanen,S.K. Potency of nonnucleoside reverse transcriptase inhibitors (NNRTIs) used in combination with other human immunodeficiency virus NNRTIs, NRTIs, or protease inhibitors. *Antimicrob. Agents Chemother.* 46, 1640-1646 (2002).

52. Piras,G., Nakade,K., Yuasa,S. & Baba,M. Three-drug combination of MKC-442, lamivudine and zidovudine in vitro: potential approach towards effective chemotherapy against HIV-1. *AIDS* 11, 469-475 (1997).

53. Carr,A. *et al.* A controlled trial of nevirapine plus zidovudine versus zidovudine alone in p24 antigenaemic HIV-infected patients. The Dutch-Italian-Australian Nevirapine Study Group. *AIDS* 10, 635-641 (1996).

54. Brennan,T.M., Taylor,D.L., Bridges,C.G., Leyda,J.P. & Tyms,A.S. The inhibition of human immunodeficiency virus type 1 in vitro by a non-nucleoside reverse transcriptase inhibitor MKC-442, alone and in combination with other anti-HIV compounds. *Antiviral Res.* 26, 173-187 (1995).

55. Esnouf,R. *et al.* Mechanism of inhibition of HIV-1 reverse transcriptase by non-nucleoside inhibitors. *Nat. Struct. Biol.* 2, 303-308 (1995).

56. Drusano,G.L. *et al.* Nucleoside analog 1592U89 and human immunodeficiency virus protease inhibitor 141W94 are synergistic in vitro. *Antimicrob. Agents Chemother.* 42, 2153-2159 (1998).

57. Pagano,P.J. & Chong,K.T. In vitro inhibition of human immunodeficiency virus type 1 by a combination of delavirdine (U-90152) with protease inhibitor U-75875 or interferon-alpha. *J. Infect Dis.* 171, 61-67 (1995).

58. Ludovici,D.W. *et al.* Evolution of anti-HIV drug candidates. Part 1: From alpha-anilinophenylacetamide (alpha-APA) to imidoyl thiourea (ITU). *Bioorg. Med. Chem. Lett.* 11, 2225-2228 (2001).

59. Ludovici,D.W. *et al.* Evolution of anti-HIV drug candidates. Part 2: Diaryltriazine (DATA) analogues. *Bioorg. Med. Chem. Lett.* 11, 2229-2234 (2001).

60. Ludovici,D.W. *et al.* Evolution of anti-HIV drug candidates. Part 3: Diarylpyrimidine (DAPY) analogues. *Bioorg. Med. Chem. Lett.* 11, 2235-2239 (2001).

Example 3 Improved anti-HIV-1 activity of compounds obtained by systemic variation of structural groups of APHS

Human immunodeficiency virus, type 1 (HIV-1) reverse transcriptase (RT) is a virus-specific RNA- and DNA-dependent DNA polymerase. This enzyme catalyzes the incorporation of deoxynucleotides 5'-triphosphate (dNTPs) into a new DNA strand, using either RNA or DNA as template. In this way, RT is responsible for the conversion of single-stranded viral RNA into double-stranded proviral DNA. This enzyme is essential for HIV-1 replication and is therefore an important target for antiretroviral (ARV) therapy. Current ARV drugs approved by the U.S. Food and Drug Administration (FDA), which inhibit the RT, are the nucleoside/nucleotide RT inhibitors (NRTI) zidovudine (AZT), lamivudine, didanosine, stavudine, zalcitabine, abacavir and tenofovir disoproxil fumarate and the non-nucleoside RT inhibitors (NNRTI) efavirenz, delavirdine and nevirapine. NRTI are phosphorylated and act competitively at the catalytic site of the RT as DNA chain-terminating analogues of the natural dNTPs [1]. NNRTI comprise many structurally diverse compounds that inhibit RT by altering either the conformation or mobility of the enzyme by binding to a specific allosteric site near the polymerase site, thereby resulting in non-competitive inhibition of the RT [2-4]. NRTI effectiveness is limited by toxicity, unfavorable pharmacokinetics and emergence of resistant viral strains [5-7]. Although NNRTI are less toxic than NRTI and have good pharmacokinetics, they rapidly select for resistant HIV-1 strains [8]. Moreover, the resistant mutations that appear in the RT enzyme frequently result in a decreased sensitivity to other RTI, thus leading to cross-resistance [8-11]. Because of these limitations, novel RTI drugs should have a good antiviral activity, be pharmacologically compatible with other anti-HIV drugs, show minimal toxicity, inhibit HIV strains that are resistant to clinically available drugs and be produced at low costs [10].
We have previously found that the selective nonsteroidal anti-inflammatory drug o-(acetoxyphenyl)hept-2-ynyl sulfide (APHS) can inhibit the replication of several HIV-1 strains in primary cells by interfering with the reverse transcription process [12] and that APHS can inhibit the replication of frequently occurring ARV-resistant HIV-1 strains [13]. The 50% inhibitory concentration ($IC_{50}$) of APHS for HIV-1, its 50% toxic concentration ($TC_{50}$) for peripheral blood mononuclear

cells and its selectivity index (SI) are 6 μM, 105 μM and 18, respectively. Since ARV should work at the nanomolar range and have a much higher SI in order to minimize daily intake and toxicity, the aim of this study was to develop new compounds by systemic variation of structural groups of APHS in order to obtain compounds with a higher antiviral activity and a lower cytotoxicity than APHS.

Experimental Section

Chemistry

Reactions were carried out under an atmosphere of dry nitrogen. All solvents were dried over sodium benzophenone ketyl and distilled prior to use. Reagents were purchased from Acros Organics. 2-Hydroxythiophenol (techn. 90%) from Lancaster, 4-hydroxythiophenol (techn 90%) and Oxone® from Aldrich were used without purification. Column chromatography was carried out with silicagel from Acros, 0.060 - 0.200 mm, pore diameter 6 nm. Chemical yields are unoptimized examples of a single preparation. The course of the reactions was followed by thin-layer chromatography (TLC) and by gas chromatography-mass spectrometry (GCMS). $^1$H- and $^{13}$C-nuclear magnetic resonance(NMR) spectra were recorded in $CDCl_3$ on a 300MHz Spectrometer. Chemical shifts are given in ppm (δ) relative to TMS as an internal standard. Coupling constants are given in Hz. Elemental analyses were carried out by Mikroanalytisches Laboratorium H.Kolbe in Mulheim-Ruhr (Germany).

APHS and the known compounds 3, 4, 5, 6, 8, 9, 10, 11, 15 and 17 up to and including 26 were synthesized according to the literature [14]. The new compounds 1, 2, 7, 12, 13, 14 and 16 were synthesized via analogous procedures.

Phenol-2(heptynylthio)-, 5, 10 and 15 are the phenolic precursors of 1, 7, 12, 13, 14 and 16. 2-Hydroxythiophenol was stirred overnight with 1.1 equivalent of bromide and 1.1 equivalent of $KHCO_3$ in DMF at room temperature. 4-Hydroxythiophenol was used as a starting material for the synthesis of 16. The bromides used were $Br\text{-}CH_2\text{-}C{\equiv}C\text{-}C_4H_9$, $Br\text{-}(CH_2)_2\text{-}O\text{-}C_4H_9$ and 1-bromoheptane. The products were purified by column chromatography on silica. Hexanes 4% EtOAc was used as eluent for 5 and phenol-2(2-heptynylthio)-; hexanes : dichloromethane = 1 : 1 was used for 10 and 15. Yields of phenol-2(2-heptynylthio)-, 5, 10 and 15 were 98%, 78%, 80% and 89% respectively.

*Benzene, 1-ethoxy-2-(2-heptynylthio)- (1)*

Phenol-2(2-heptynylthio)- was stirred with 1.1 equivalent of ethyliodide and $K_2CO_3$ in DMF at room temperature during 20 hours. After addition of water the water layer was extracted three times with pentane. The combined pentane layers were washed with water and dried over $MgSO_4$. The crude product was purified by column chromatography (silicagel / hexanes). Yield 92%.
$^1$H NMR: 7.38-7.41 (dd, 1H, $^3J_{HH}$ = 7.6 and $^4J_{HH}$ =1.5, ArH), 7.15-7.21 (dt, 1H, $^3J_{HH}$ = 7.7 and $^4J_{HH}$ = 1.8, ArH), 6.88-6.97 (dt, 1H, $^3J_{HH}$ = 7.7 and $^4J_{HH}$ =1.2, ArH), 6.80-6.87 (dd, 1H, $^3J_{HH}$ =8.0 and $^4J_{HH}$ = 1.1, ArH), 4.08 (q, 2H, $^3J_{HH}$ = 7.0, $OCH_2$), 3.65 (t, 2H, J = 2.2, $SCH_2$), 2.06-2.20 (m, 2H, $C{\equiv}C\text{-}CH_2$), 1.30-1.54. (m, 7H, $2xCH_2$ + $CH_3$), 0.88 (t, 3H, $^3J_{HH}$ = 7.2, $CH_3$). $^{13}$C NMR: 157.0, 130.3, 127.8, 124.3, 121.1, 111.7, 84.0, 75.8, 64.4, 31.0, 22.1, 21.3, 18.7, 15.0, 13.9.

*Acetic acid, trifluoro-, 2-(2-butoxyethylthio)phenylester (7)*

Compound 5 was stirred overnight with 1.1 equivalent of trifluoroacetic anhydride and pyridine in dichloromethane at room temperature. Water was added and the water layer was extracted three times with dichloromethane. The combined dichloromethane layers were washed four times with a 2N aqueous HCl solution and finally with water and dried over $MgSO_4$. The product was purified by flash distillation. Yield 96%.
$^1$H NMR: 7.48-7.56 (m, 1H, ArH), 7.22-7.30 (m, 2H, ArH), 7.08-7.16 (m, 1H, ArH), 3.55 (t, 2H, $^3J_{HH}$ = 6.7, $CH_2$), 3.40 (t, 2H, $^3J_{HH}$ = 6.7, $CH_2$), 3.04 (t, 2H, $^3J_{HH}$ = 6.7, $CH_2$), 1.46-1.58 (m, 2H, $CH_2$). 1.28-1.44 (m, 2H, $CH_2$), 0.90 (t, 3H, $^3J_{HH}$ = 7.3, $CH_3$). $^{13}$C NMR: 155.7 (q, $^2J_{CF}$ =43.6), 148.7, 132.4, 129.2, 128.3, 128.2, 121.8, 114.9 (q, $^1J_{CF}$ = 285.0), 71.1, 69.4, 33.7, 31.9, 19.4, 13.9. GCMS: M = 322. Elemental analysis: Found, C 52.29, H 5.40, F 17.46, O 14.76, S 9.91 %. Calcd., C 52.17, H 5.32, F 17.68, O 14.89, S 9.95 %.

*Acetic acid, trifluoro-, 2-(heptylthio) phenylester (12)*

12 was synthesized from 10 in a similar way. The product was purified by flash distillation. Yield 93%.
NMR: 7.45-7.54 (m, 1H, ArH), 7.24-7.36 (m, 2H, ArH), 7.12-7.21 (m, 1H, ArH), 2.89 (t, 2H, $^3J_{HH}$ = 7.4, $SCH_2$), 1.56-1.72 (m, 2H, $CH_2$), 1.20-1.52 (m, 8H, $4xCH_2$), 0.91 (t, 3H, $^3J_{HH}$ = 6.8, $CH_3$). $^{13}$C NMR: 155.8 (q, $^2J_{CF}$ = 43.5), 148.5, 131.8,

130.0, 128.1, 127.8, 121.8, 114.9 (q, $^1J_{CF}$ = 284.0), 33.9, 31.9, 29.2, 29.0, 28.9, 22.8, 14.2. GCMS: M = 320

*Phenol, 2-(heptylthio)-, valerate (13)*

13 was synthesized from 10 in a similar way using 1.1 equivalent of valeric anhydride. The reaction took 36 hours to completion. The product was purified by column chromatography (silicagel / hexanes : dichloromethane = 1: 1). Yield 91%.
$^1$H NMR: 7.34-7.42 (m, 1H, ArH), 7.16-7.24 (m, 2H, ArH), 7.02-7.10 (m, 1H, ArH), 2.87 (t, 2H, $^3J_{HH}$ = 7.2, $SCH_2$), 2.63 (t, 2H, $^3J_{HH}$ = 7.2, $COCH_2$), 1.74-1.88 (m, 2H, $CH_2$), 1.58-1.71 (m, 2H, $CH_2$), 1.20-1.58 (m, 10H, 5x $CH_2$), 1.00 (t, 3H, $^3J_{HH}$ = 7.2, $CH_3$), 0.91 (t, 3H, $^3J_{HH}$ = 6.6, $CH_3$). $^{13}$H NMR: 172.0, 149.6, 130.4, 130.2, 127.0, 126.6, 122.9, 34.1, 33.3, 32.0, 29.3, 29.1, 29.0, 27.2, 22.8, 22.5, 14.3, 14.0. GCMS: M = 308. Elemental analysis: Found, C 70.17, H 9.12, O 10.30, S 10.36 %. Calcd., C 70.08, H 9.15, O 10.39, S 10.37 %.

*Phenol, 2-(heptylthio)-, benzoate (14)*

14 was prepared by stirring overnight 1.0 equivalent of 10 with 1.1 equivalent of benzoylchloride and 1.1 equivalent of triethylamine in diethylether. The reaction mixture was diluted with ether and the organic layer was washed five times with a 2N aqueous HCl solution and subsequently two times with water. The product was purified by column chromatography (silicagel / hexanes : dichloromethane = 1:1). Yield 98%.
$^1$H NMR: 8.31 (d, 2H, $^3J_{HH}$ = 7.8, ArH). 7.66 (t, 1H, $^3J_{HH}$ = 7.2, ArH), 7.54 (t, 2H, $^3J_{HH}$ = 7.6, ArH), 7.43-7.50 (m, 1H, ArH), 7.21-7.33 (m, 3H, ArH), 2.91 (t, 2H, $^3J_{HH}$ = 7.2, $SCH_2$), 1.55-1.75 (m, 2H, $CH_2$), 1.20-1.55 (m, 8H, $4xCH_2$), 0.93 (t, 3H, $^3J_{HH}$ = 6.9, $CH_3$). $^{13}$C NMR: 164.9, 149.8, 133.9, 130.7, 130.6, 130.2, 129.7, 128.9, 127.1, 126.8, 123.1, 33.4, 32.0, 29.3, 29.1 (2x), 22.9, 14.4. GCMS: M = 328 Elemental analysis: Found, C 73.29, H 7.45, O 9.62, S 9.78 %. Calcd., C 73.13, H 7.36, O 9.74, S 9.76 %.

*Phenol, 4-(heptylthio)-, acetate (16)*

16 was synthesized from 15 and 1.1 equivalent of acetic anhydride similar to 7. The product was purified by column chromatography (silicagel / dichloromethane). Yield 96%.
NMR: 7.32(d, 2H, $^3J_{HH}$ = 8.4, ArH), 7.00 (d, 2H, $^3J_{HH}$ = 8.7, ArH), 2.87 (t, 2H, $^3J_{HH}$ = 7.2, $SCH_2$), 2.24 (s, 3H, $COCH_3$), 1.58-1.70 (m, 2H, $CH_2$), 1.20-1.50 (m, 8H, $4xCH_2$), 0.89 (t, 3H, $^3J_{HH}$ = 6.8, $CH_3$). $^{13}$C NMR: 169.4, 149.1, 134.5, 130.4, 122.2, 34.4, 32.0, 29.4, 29.1, 29.0, 22.8, 21.2, 14.3. GCMS: M = 266. Elemental analysis: Found, C 67.54, H 8.28, O 12.11, S 12.03 %. Calcd., C 67.63, H 8.32, O 12.01, S 12.04 %.

*Compounds stocks*

APHS and APHS derivatives were diluted in aliquots in 100% ethanol, topped with argon gas and stored at -20 °C. The concentration of ethanol during incubations never exceeded 0.1%. At this concentration, ethanol did not affect HIV-1 replication or cellular viability (data not shown).

*Isolation of primary cells*

Donor peripheral blood mononuclear cells (PBMC) were isolated from heparinized blood from HIV-1-, HIV-2-, and hepatitis B-seronegative donors and obtained on Ficoll-Isopaque density gradients. To prepare a PBMC mixed batch, PBMC isolated from six donors were pooled together in RPMI 1640 medium (Gibco, Life Technologies Ltd., Paisley, Scotland) supplemented with 10% dimethyl sulfoxide (DMSO) (Merck, Darmstadt, Germany), 20% fetal calf serum (FCS) (Life Technologies) and 10 μg/ml gentamycin (Life Technologies) and frozen at -140 °C. Cells were thawed and cultured for 4 days prior to the experiment in medium RPMI 1640 medium supplemented with 10% FCS, and 10 μg/ml gentamycin containing 2 μg/ml lectin from *Phaseolus vulgaris* (PHA) (Sigma Chemie, Zwijndrecht, The Netherlands) at 37°C and 5% $CO_2$.

*HIV-1 infection of PBMC*

PHA-stimulated PBMC were washed twice and incubated for 7 days in a concentration of $5 \times 10^5$ cells/ml with HIV-$I_{Ba-L}$, at a multiplicity of infection (MOI) of 0.0025 (input virus), in the presence of APHS or related compounds and 10 U/ml recombinant interleukine-2 (IL-2) (Roche Diagnostics GmbH, Mannheim, Germany), at 37°C and 5% $CO_2$. To correct for the input virus, an extra control consisting of medium containing the same amount of input virus as added to the cells was included in the experiment. The amount of p24 in this control was subsequently subtracted from the

p24 values of the samples in order to obtain the exact amount of p24 produced by the cells.

*p24-core antigen quantification by ELISA*

After 7 days incubation, samples of the supernatants were collected, inactivated by addition of Empigen (Calbiochem, La Jolla, CA, USA) and by heat inactivation at 56°C for 30 min. p24-core antigen concentration was determined by an enzyme linked immunoabsorbent assay (ELISA) (AMPAK™, DAKO, Cambridgeshire, UK) as described previously 15,16. The optical density values were converted into p24 concentration (ng/ml) with the use of a calibration curve made by serial dilutions of recombinant p24 protein (NIBSC, UK) that was submitted to the same treatment as the samples.

Determination of viability of PBMC

PHA-stimulated PBMC were washed twice and incubated in a concentration of $5 \times 10^5$ cells/ml with increasing concentrations of APHS or related compounds for 7 days at 37°C and 5% $CO_2$ Afterwards the metabolic activity of these cells was assessed by a cellular viability assay as described previously [17]. Shortly, 150 μg/ml tetrazollium salt 3-(4,5-dimethylthiazol-2-yl)-2,5-diphenyltetrazolliumbromide (MTT) (Sigma) is added to the cells. During the subsequent 2h incubation period metabolic active cells convert MTT into blue formazan crystals. Afterwards, ¾ of the supernantant was gently removed and substituted by stop buffer consisting of 90% 2-propanol, 10% triton X-100 and 0.4% HCl (Merck, Darmstadt, Germany). When all formazan crystals were dissolved optical density was measured at 550 nm and using 630 nm as reference.

*Determination of $IC_{50}$ and $TC_{50}$ of the compounds*

The ability of each compound to decrease p24 antigen and cellular viability were expressed as the 50% inhibitory concentration ($IC_{50}$) and the 50% toxic concentration ($TC_{50}$), respectively. $IC_{50}$ and $TC_{50}$ were calculated using the computer software program CalcuSyn for Windows (Biosoft, Cambridge, UK) according to the method of Chou and Talalay [18,18,19]. This program uses the median-effect equation to produce dose-effect curves:

$$f_a = 1/[1 + (D_m/D)^m] \quad \text{[equation 1]}$$

where $f_a$ represents fraction affected by the dose (reduction of p24 or cellular viability at a certain drug concentration expressed in decimals), $D_m$ is the median effect dose (same as $IC_{50}$ or $TC_{50}$), $D$ is the dose of the drug and m is the sigmoidicity coefficient of the dose-effect curve. Data was accepted when the linear correlation coefficient of the median-effect plot based on experimental data was > 0.90. The selectivity index (SI), which represents the concentration range where a drug is effective without being toxic, was calculated by the ratio $TC_{50}/IC_{50}$.

Results

Antiviral activity and cytotoxicity of APHS derivatives

By systematic variation of structural groups of APHS several derivatives were synthesized and the anti-HIV-1 activity and cytotoxicity were determined (Table 3.1). When the acetate group at position 1' was substituted by an ethyl (compound 1) or butyl (compound 2) group, the antiviral activity decreased (higher $IC_{50}$). In addition, the cytotoxicity of the compounds also decreased (higher $TC_{50}$) and as a consequence, the SI of these compounds decreased approximately 8 times. When the 2-heptynylthio group at position 2' was substituted by an hexanoic group (compound 4), the antiviral activity and cytotoxicity decreased. When both the 2-heptynylthio and the acetate groups were substituted by an hexanoic and an hydroxyl group, respectively (compound 3), the antiviral activity and cytotoxicity also decreased. When the 2-heptynylthio group was substituted by a 2-butoxyethyl group (compound 6), the antiviral activity and cytotoxicity decreased. When the 2-heptynylthio group was substituted by a 2-butoxyethyl group and, simultaneously, the acetate group was substituted by an hydroxyl group or by a trifluoro-acetic acid (compounds 5 and 7, respectively), the antiviral activity and cytotoxicity also decreased.
Removal of the 2-heptynylthio group at position 2' and substitution by an heptylthio group at position 4' (compound 16) resulted in an increase in antiviral activity (2 μM), but also in cytotoxicity. When this change was made simultaneously with a substitution of the acetate group by an hydroxyl group (compound 15) both the antiviral activity (3 μM) and cytotoxicity increased. When the 2-heptynylthio group was substituted by an heptylthio group and the acetate group

was substituted by a trifluoro-acetic acid, a valerate, or a benzoate group (compounds 12, 13 or 14, respectively), the antiviral activity decreased and the cytotoxicity decreased for compounds 12 and 13 and increased for compound 14 when compared to APHS. Substitution of the 2-heptynylthio group by an octylthio, heptylthio or hexylthio group (compounds 9, 11 and 18, respectively) resulted in a gradual increase in antiviral activity (20, 10 and 3 μM, respectively) and in a decrease in cytotoxicity (135, 48 and 119 μM, respectively). These substitutions resulted in an SI of 7, 5, and 48, respectively. These results seem to indicate that a shorter carbon chain at position 2' correlates with in an increase in antiviral activity. Substituting the acetate group by an hydroxyl group and, simultaneously, substituting the 2-heptynylthio group by an octylthio, heptylthio or hexylthio group (compounds 8, 10 and 17, respectively), resulted in an increase in cytotoxicity. Compounds 8 and 10 showed less antiviral activity than APHS while compound 17 showed an improvement in antiviral activity ($IC_{50}$ of 4 μM).
Since compound 18 showed the highest SI, derivatives of this compound were made by systematic shortening of the carbon chain (Table 3.2). The hexylthio group was substituted by a pentylthio, butylthio, propylthio or ethylthio group (compounds 20, 22, 24 and 26, respectively). Surprisingly, all these compounds showed worse antiviral activity than compound 18, although the cytotoxicity gradually decreased with the shortening of the carbon chain. Similarly, when the acetate group of these compounds was substituted by an hydroxyl group (19, 21, 23 and 25), a decrease in cytotoxicity seemed to correlate with the shortening of the carbon chain. Interestingly, these compounds showed improved antiviral activity (0.9, 3, 5 and 4 μM, respectively) when compared to APHS. The most potent antiviral drug tested was compound 19 with an SI of 159.

In order to increase the antiviral activity and decrease the cytotoxicity of APHS, new compounds were synthesized by systemic variation of structural groups of APHS. Twenty one substitutions resulted in a decrease in cytotoxicity but only eight substitutions resulted in an increase in antiviral activity. Substitution of the acetate group of APHS at position 1' for an ethyl (1) or a butyl (2) group or substitution of the acetate group of compound 11 at position 1' for a trifluoro-acetic acid, a valerate, or a benzoate group (compounds 12, 13 or 14, respectively) resulted in a decrease of antiviral activity, which suggest that the size and conformation of the group at this position is very important for the antiviral activity of the compounds. Interestingly, substitution of the acetate group by an hydroxyl group resulted in both a decrease or an increase in antiviral activity, depending on the length of the hydrophobic chain at position 2'. However, the compound with the best antiviral activity (19) exhibited an hydroxyl group instead of an acetate group (20), which indicates that an hydroxyl group is sufficient or even preferable than an acetate group at this position.
When the hydrophobic 2-heptynylthio group of APHS at position 2' was substituted by an hexanoic group (4) or by an 2-butoxyethyl group (6) the antiviral activity decreased, which suggests that the substitution of the alkyne by a carboxylic acid or by a ether will compromise antiviral activity. Removal of the 2-heptynylthio group of APHS at position 2' and substitution by an heptylthio group at position 4' (16) resulted in an increase in antiviral activity but also in an increase in cytotoxicity. However, more substitutions at this and other positions will have to be done before further conclusions can be drawn. Substitution of the 2-heptynylthio group of APHS by an heptylthio group (11) resulted in a decrease of antiviral activity, which seems to indicate that the antiviral activity is related with the rigid triple bound in this group. Interestingly, substitution of the 2-heptynylthio group of APHS by an hexylthio (18) or by an octylthio (9) group resulted in an increase or in further decrease of antiviral activity, respectively, suggesting that the size of this carbon chain and not the rigid triple bound is essential for the antiviral activity of these compounds. The same increase in anti-HIV activity was observed when, simultaneously, the acetate group was substituted by an hydroxyl group (17). Since compounds with a shorter carbon chain seem to have a better antiviral activity, the effect of systematic shortening of the carbon chain of compounds 18 and 17 on HIV-1 replication was studied. Interestingly, the same trend was not observed for this new set of compounds. Indeed, compounds with a pentylthio group (compounds 20 and 19) showed better antiviral activity than compounds with an ethylthio group (compounds 26 and 25). This suggests that the carbon chain with five carbons has the optimum size for the best antiviral activity. Importantly, although two APHS derivatives (compounds 19 and 20) possessed a pentylthio group, only the one with an hydroxyl group instead of the acetate group (19) had a better antiviral activity than APHS. This suggests that the antiviral activity of all APHS derivatives tested results from a an interplay between both side groups.
Preliminary results originated by the computational program docking which simulates the binding of APHS derivatives to a three-dimensional structure of the HIV-1 RT, predicted that four of the APHS derivatives (compounds 18, 19, 24 and 26) could bind to the same hydrophobic pocket located at positions 369-412 on the connection domain of the HIV-1 RT (data not shown). Interestingly, the aa residues surrounding this hydrophobic pocket were mainly hydrophobic non-ligand residues involved in hydrophobic contacts. However, the APHS derivatives formed hydrogen bounds through their acetate or hydroxyl groups with two hydrophilic aa :Thr 397 and Gln 394. Although data obtained by docking only gives an indication of possible binding sites, it suggests that decreasing the hydrophobicity of APHS derivatives will probably improve antiviral activity.
Most of the substitutions resulted in a decrease in cytotoxicity. Smaller compounds (compounds 23, 24, 25 and 26) were 2- to 4-fold less cytotoxic than APHS. Removal of the 2-heptynylthio group of APHS at position 2' and substitution by an heptylthio group at position 4' (compounds 16 and 15) resulted in about 10-fold increase in cytotoxicity. Interestingly, the most hydrophilic compounds are also less cytotoxic. Substitution of the 2-heptynylthio group of APHS by an hexanoic acid group (4) or by a 2-butoxyethyl group (6) resulted in a 3- or 5- fold decrease of cytotoxicity. These substitutions of an hydrophobic group by a more hydrophilic group are in agreement with quantitative structure-activity relationship (QSAR) analysis of all these compounds (data not shown). This analysis indicated that the toxicity of the compounds is caused by the high degree of hydrophobicity. Before more conclusions can be drawn, a larger group of compounds have to be developed and tested. These compounds should contain more variations on physicochemical parameters reflecting steric, electrostatic and hydrophobic properties. The new compounds should also contain variations on the position of the side groups (1' and 2',3' or 4').

Table 3.1. Effect of APHS and its derivatives on HIV-$1_{Ba\text{-}L}$ replication and cellular viability in PBMC. Chemical structures of APHS and its derivatives are also shown.

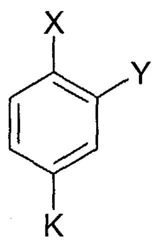

| Compound | X | Y | K | $IC_{50}$[a] | $TC_{50}$[b] | SI[c] |
|---|---|---|---|---|---|---|
| APHS | $OC=OCH_3$ | $SCH_2C \equiv CC_4H_9$ | - | 6 ± 2 | 105 ± 15 | 18 |
| 1 | $OC_2H_5$ | $SCH_2C \equiv CC_4H_9$ | - | 68 ± 22 | 245 ± 17 | 4 |
| 2 | $OC_4H_9$ | $SCH_2C \equiv CC_4H_9$ | - | 65 ± 24 | 191 ± 6 | 3 |
| 3 | OH | $S(CH_2)_5COOH$ | - | 41 ± 8 | 442 ± 67 | 11 |
| 4 | $OC=OCH_3$ | $S(CH_2)_5COOH$ | - | 31 ± 7 | 281 ± 30 | 7 |
| 5 | OH | $S(CH_2)_2OC_4H_9$ | - | 26 ± 16 | 199 ± 12 | 4 |
| 6 | $OC=OCH_3$ | $S(CH_2)_2OC_4H_9$ | - | 51 ± 25 | 530 ± 105 | 10 |
| 7 | $OC=OCF_3$ | $S(CH_2)_2OC_4H_9$ | - | 22 ± 12 | 168 ± 19 | 5 |
| 8 | OH | $SC_8H_{17}$ | - | 8 ± 1 | 76 ± 29 | 9 |
| 9 | $OC=OCH_3$ | $SC_8H_{17}$ | - | 20 ± 10 | 135 ± 22 | 7 |
| 10 | OH | $SC_7H_{15}$ | - | 61 ± 6 | 89 ± 27 | 1 |
| 11 | $OC=OCH_3$ | $SC_7H_{15}$ | - | 10 ± 2 | 48 ± 14 | 5 |
| 12 | $OC=OCF_3$ | $SC_7H_{15}$ | - | 55 ± 19 | 125 ± 47 | 2 |
| 13 | $OC=OC_4H_9$ | $SC_7H_{15}$ | - | 43 ± 2 | 110 ± 43 | 3 |
| 14 | $OC=OC_6H_5$ | $SC_7H_{15}$ | - | 16 ± 2 | 44 ± 5 | 3 |
| 15 | OH | - | $SC_7H_{15}$ | 3 ± 1 | 8 ± 3 | 3 |
| 16 | $OC=OCH_3$ | - | $SC_7H_{15}$ | 2 ± 1 | 14 ± 2 | 7 |
| 17 | OH | $SC_6H_{13}$ | - | 4 ± 1 | 80 ± 27 | 22 |
| 18 | $OC=OCH_3$ | $SC_6H_{13}$ | - | 3 ± 1 | 119 ± 26 | 48 |

[a] $IC_{50}$ represents 50% inhibitory concentration.

[b] $TC_{50}$ represents 50% toxic concentration.

[c] The SI represents the $TC_{50}$ / $IC_{50}$ ratio.

Results are the average of at least three independent experiments.

Table 3.2. Effect of APHS derivatives on $HIV\text{-}1_{Ba\text{-}L}$ replication and cellular viability in PBMC. Chemical structures of APHS derivatives are also shown.

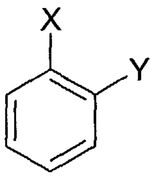

| Compound | X | Y | $IC_{50}$[a] | $TC_{50}$[b] | SI[c] |
|---|---|---|---|---|---|
| 19 | OH | $SC_5H_{11}$ | 0.9 ± 0.4 | 149 ± 51 | 159 |
| 20 | OC=OCH3 | $SC_5H_{11}$ | 11 ± 4 | 102 ± 5 | 10 |
| 21 | OH | $SC_4H_9$ | 3 ± 1 | 89 ± 11 | 27 |
| 22 | OC=OCH3 | $SC_4H_9$ | 36 ± 5 | 160 ± 40 | 5 |
| 23 | OH | $SC_3H_7$ | 5 ± 2 | 225 ± 49 | 48 |
| 24 | OC=OCH3 | $SC_3H_7$ | 34 ± 6 | 307 ± 58 | 9 |
| 25 | OH | $SC_2H_5$ | 4 ± 1 | 373 ± 87 | 86 |
| 26 | OC=OCH3 | $SC_2H_5$ | 28 ± 15 | 411 ± 58 | 15 |

[a] $IC_{50}$ represents 50% inhibitory concentration.

[b] $TC_{50}$ represents 50% toxic concentration.

[c] The SI represents the $TC_{50}$ / $IC_{50}$ ratio.

Results are the average of at least three independent experiments.

References with example 3

1. St Clair,M.H. *et al.* 3'-Azido-3'-deoxythymidine triphosphate as an inhibitor and substrate of purified human immunodeficiency virus reverse transcriptase. *Antimicrob. Agents Chemother.* 31, 1972-1977 (1987).
2. Esnouf,R.M. *et al.* Unique features in the structure of the complex between HIV-1 reverse transcriptase and the bis(heteroaryl)piperazine (BHAP) U-90152 explain resistance mutations for this nonnucleoside inhibitor. Proc. *Natl. Acad. Sci. U. S. A* 94, 3984-3989 (1997).
3. Ding,J. *et al.* Structure of HIV-1 RT/TIBO R 86183 complex reveals similarity in the binding of diverse nonnucleoside inhibitors. *Nat. Struct. Biol.* 2, 407-415 (1995).
4. Ren,J. *et al.* High resolution structures of HIV-1 RT from four RT-inhibitor complexes. *Nat. Struct. Biol.* 2, 293-302 (1995).
5. Richman,D.D. *et al.* The toxicity of azidothymidine (AZT) in the treatment of patients with AIDS and AIDS-related complex. A double-blind, placebo-controlled trial. *N. Engl. J. Med.* 317, 192-197 (1987).
6. Richman,D.D. Resistance of clinical isolates of human immunodeficiency virus to antiretroviral agents. *Antimicrob. Agents Chemother.* 37, 1207-1213 (1993).
7. Terasaki,T. & Pardridge,W.M. Restricted transport of 3'-azido-3'-deoxythymidine and dideoxynucleosides through the blood-brain barrier. *J. Infect. Dis.* 158, 630-632 (1988).
8. Deeks,S.G. International perspectives on antiretroviral resistance. Nonnucleoside reverse transcriptase inhibitor resistance. *J. Acquir. Immune. Defic. Syndr.* 26 Suppl 1, S25-S33 (2001).
9. de Jong,J.J. *et al.* Insertion of two amino acids combined with changes in reverse transcriptase containing tyrosine-215 of HIV-1 resistant to multiple nucleoside analogs. *AIDS* 13, 75-80 (1999).

10. Richman,D.D. HIV chemotherapy. *Nature* 410, 995-1001 (2001).

11. Loveday,C. International perspectives on antiretroviral resistance. Nucleoside reverse transcriptase inhibitor resistance. *J. Acquir. Immune. Defic. Syndr.* 26 Suppl 1, S10-S24 (2001).

12. Pereira,C.F. *et al.* Aspirin-like molecules that inhibit human immunodeficiency virus-1 replication. *Antivir. Res., in press* (2003).

13. Pereira,C.F. *et al.* APHS can act synergistically with clinically available HIV-1 reverse transcriptase and protease inhibitors and is active against several drug-resistant HIV-1 strains *in vitro. J. Antimicrob. Chemoth., resubmitted.*

14. Kalgutkar,A.S., Kozak,K.R., Crews,B.C., Hochgesang,G.P.J. & Marnett,L.J. Covalent modification of cyclooxygenase-2 (COX-2) by 2-acetoxyphenyl alkyl sulfides, a new class of selective COX-2 inactivators. *J. Med. Chem.* 41, 4800-4818 (1998).

15. McKeating,J.A., McKnight,A. & Moore,J.P. Differential loss of envelope glycoprotein gp 120 from virions of human immunodeficiency virus type 1 isolates: effects on infectivity and neutralization. *J. Virol.* 65, 852-860 (1991).

16. Moore,J.P., McKeating,J.A., Weiss,R.A. & Sattentau,Q.J. Dissociation of gp120 from HIV-1 virions induced by soluble CD4. *Science* 250, 1139-1142 (1990).

17. Pauwels,R. *et al.* Rapid and automated tetrazolium-based colorimetric assay for the detection of anti-HIV compounds. *J. Virol. Methods* 20, 309-321 (1988).

18. Chou,T.C. & Hayball,M.P. (BIOSOFT, Cambridge,UK,1996).

19. Chou,T.C. & Talalay,P. Quantitative analysis of dose-effect relationships: the combined effects of multiple drugs or enzyme inhibitors. *Adv. Enzyme Regul.* 22, 27-55 (1984).

Example 4 Spectrum of antiviral activity of the aspirin-like molecule *o*-(acetoxyphenyl)hept-2-ynyl sulfide (APHS)

We have previously found that the non-steroidal anti-inflammatory drug o-(acetoxyphenyl)hept-2-ynyl sulfide (APHS) can inhibit human immunodeficiency virus, type 1 (HIV-1) replication by interfering with the reverse transcription process [1]. HIV-1 is a member of the retroviridae family. Retroviruses are RNA viruses, which possess a reverse transcriptase (RT) enzyme, which is a RNA- and DNA-dependent DNA polymerase (DP), that transcribes the viral RNA into proviral DNA. This proviral DNA can be inserted into the cellular genome by the viral integrase. Once the virus is integrated into the cellular genome it can stay latent for many years until the cell is stimulated and viral transcription starts.

Some antiviral compounds have a broad spectrum of action while others are more specific [2]. The pyrophosphate analogue phosfonoformic acid (foscarnet, PFA) is a broad-spectrum DP inhibitor that is active against several DNA and RNA viruses, including HSV-1 and HIV-1,and acts by interfering with the exchange of pyrophosphate from deoxynucleoside triphosphate during viral replication by binding to a site on the HSV DP or HIV-1 RT [3,4]. Acyclic nucleoside phosphonates are chain terminators which are active against HIV, FIV, HSV, adenovirus, papillomaviruses and hepatitis B virus [5-7]. Receptor antagonists such as bicyclams inhibit cell-virus fusion and can inhibit HIV, SIV and FIV replication [2]. Most of the non-nucleoside reverse transcriptase inhibitors (NNRTI) are very specific; for instance they inhibit the replication of HIV-1, but not that of HIV-2[8].

The aim of this study was to determine the antiviral specificity of APHS against viruses representative of different classes. Feline immunodeficiency virus (FIV) is also a retrovirus, which resembles HIV-1 in its morphological, physical, biochemical and pathological characteristics and is therefore an adequate model to study the effect of antiviral therapy in vivo [9,10]. Similarities between the RTs of FIV and HIV-1 have been demonstrated with respect to template specificity and magnesium requirement [11]. Respiratory syncytium virus (RSV) is a negative (-) stranded RNA virus, which belongs to the family paramyxoviridae. The RSV core contains an RNA replicase, which is an RNA-dependent RNA polymerase. When released into the cell cytoplasm, this RNA polymerase makes a complementary copy of the genome, which is (+) stranded. This complementary strand acts as a template for genome synthesis (replication). Simultaneously, a series of (+) strands are produced, which act as mRNAs. These are translated into proteins needed to assemble new virions or to act as templates for the synthesis of (-) strands that will be incorporated into new virions. Mouse hepatitis virus (MHV) is a positive (+) stranded RNA virus, which belongs to the family coronaviridae. The MHV genome serves as an mRNA for a polyprotein from which, by proteolytic cleavages the various subunits of the viral RNA-dependent RNA polymerase are derived. The coronaviral RNA polymerase directs the synthesis of both genome-length and subgenomic negative-stranded RNAs, which in turn serve as templates for the synthesis of genomic RNA and subgenomic mRNAs, respectively.

Herpes simplex virus-1 (HSV-1) is a double-stranded DNA virus, which belongs to the family herpesviridae and subfamily $\alpha$-herpesviridae. After virus entry into the cytoplasm, the nucleocapsid is transported to the nuclear pores where the viral DNA is released into the nucleus. The HSV DNA forms a circular molecule, which acts as the template for replication. Early gene expression results in the expression of enzymes involved in nucleic acid metabolism such as thymidine kinase (TK) and proteins essential for DNA synthesis such as DNA replicase, which is a DNA-dependent

DP. The DP catalyses the viral DNA synthesis. New progeny virus DNA is synthesized off the DNA genome of the input parental virus. New progeny DNA acts as a template for the synthesis of more genomes for new virus particles and for transcription of late virus mRNA that encode mainly viral structural proteins.

Materials and Methods

*Cells*

Crandell feline kidney (CrFK) cells were maintained in Dulbecco's modified Eagle's medium (DMEM; Gibco, Invitrogen, Paisley, Scotland), supplemented with 5% fetal calf serum (FCS; Invitrogen) and antibiotics. Human epithelial (HEp-2) cells were cultured as monolayers in Iscove's modified Dulbecco's medium (IMDM; Invitrogen), supplemented with 5% FCS and 10 μg/ml gentamycin (Invitrogen) and maintained at 37 °C and 5% $CO_2$. Mouse L cells were cultured in DMEM containing 10% FCS, 100 IU/ml penicillin and 100 μg/ml streptomycin (DMEM/10). Human foreskin fibroblasts (HFF) were cultured as monolayers in IMDM supplemented with 10% FCS and 10 μg/ml gentamycin and maintained at 37 °C and 5% $CO_2$.

*Virus*

A virus stock of the Dutch isolate FIV UT-113 was prepared from a culture of persistently infected CrFK cells. RSV serotype A was propagated and titrated in HEp-2 cells. RSV stock contained $2.4 \times 10^7$ plaque-forming units (PFU)/ml. MHV-A59 was propagated in Moloney sarcoma virus-transformed Sac(-) cells and plaque titrated on mouse L cells as described previously [12]. MHV stock contained $5.3 \times 10^8$ PFU/ml. The HSV-1 strains KOS (wild-type) and the KOS-derived acyclovir (ACV)-resistant AraA$^r$8 and ACV- and or phosphonoacetic acid (PAA)-resistant PAA$^r$5, which contain mutations in the DP gene [13-15] were kindly provided by D. M. Coen (Harvard Medical School, Boston, MA, USA). The ACV-resistant clinical isolate which contains mutations in the TK gene 98.14742-PE/1 [16] was a gift of M. Aymard (Université Claude Bernard, Lyon, France). HSV-1 stocks were grown in Vero cells (African green monkey kidney) and the infectious titer was determined by plaque assay in Vero cells as described previously [17].

Compounds

APHS and the APHS derivative phenol, 2-(pentylthio) (APHS-d) were synthesized at the Department of Metal-Mediated Synthesis of the University of Utrecht. Synthesis details have been described elsewhere (Pereira C.F. *et al.* submitted for publication). All compounds tested were diluted in aliquots in 100% ethanol, topped with argon gas and stored at -20°C. The concentration of ethanol during incubations never exceeded 0.1%. At this concentration, ethanol did not affect virus replication or cellular viability (data not shown).

*Determination of cellular viability*

CrFK cells grown in 96-well plates to confluent monolayers were incubated with fivefold dilutions of the antiviral drugs (ranging from 100 to 0.024 μM) and after 6 days of incubation at 37 °C and 5% $CO_2$, a cellular viability assay was performed on these cells. HEp-2 cells were seeded in 96-wells plates in the presence of APHS for 4, 5 and 6 days. Concentrations of HEp-2 cells were determined by microscopic observation in order to have a confluent monolayer at the day of measurement. Afterwards a cellular viability assay was performed on these cells. HFF cells were seeded and incubated for 2 days at 37 °C and 5% $CO_2$ Subsequently, the medium was removed and replaced by medium containing increasing concentrations of APHS. After 24h of incubation the medium was aspirated and the HFF monolayer was overlayed with baby hamster kidney (BHK) cells and the culture was incubated for 24h at 37 °C and 5% $CO_2$ Afterwards, a cellular viability assay was performed on these cells.
The metabolic activity of the above mentioned cells was assessed by a cellular viability assay as described previously[18]. Shortly, 150 μg/ml tetrazollium salt 3-(4,5-dimethylthiazol-2-yl)-2,5-diphenyltetrazolliumbromide (MTT; Sigma) is added to the cells. During the subsequent 2h incubation period metabolic active cells convert MTT into blue formazan crystals. Subsequently, ¾ of the supernatant was gently removed and substituted by stop buffer consisting of 90% 2-propanol, 10% triton X-100 and 0.4% HCl (Merck, Darmstadt, Germany). When all formazan crystals were dissolved optical density was measured at 550 nm using 630 nm as reference wavelength.

FIV infection of CrFK cells

CrFK cells grown in 96-well plates to confluent monolayers were preincubated for 1h with fourfold dilutions of APHS or APHS-d (ranging from 100 to 0.024 μM) and subsequently infected with 100 50% tissue culture infective

doses ($TCID_{50}$) of FIV UT-113. After 2h, CrFK cells were rinsed twice with PBS and the cultures were maintained in the presence of different dilutions of APHS or APHS-d. After 6 days of incubation FIV replication was measured by quantification of the p24 viral core antigen in the cell culture supernatant by an FIV p24 enzyme-linked immunoabsorbent assay as described previously [19].

*RSV infection of HEp-2 cells*

RSV infection of the HEp-2 cell line was performed as described elsewhere (Hament J-M et al., submitted for publication). Briefly, HEp-2 cells were recovered from the culture flasks by trypsinization. Concentrations of HEp-2 cells were determined by microscopic observation in order to have a confluent monolayer at the day of measurement. HEp-2 cells were incubated with APHS and infected with different dilutions of RSV in order to have a good infection at the day of measurement. After 4, 5 and 6 days of incubation at 37 °C and 5% $CO_2$ HEp-2 cell monolayers were washed three times with phosphate buffered saline (PBS) and cells were recovered by trypsinization. The cells were washed with $PBS^{2+}$ (PBS containing 0.15 mM $Ca^{2+}$ and 0.5 mM $Mg^{2+}$) and incubated with murine-anti-RSV monoclonal antibody conjugated to fluorescein isothiocyanate (FITC) (DAKO, Cambridgeshire, UK) for 15 minutes at 37 °C. Afterwards, cells were washed and resuspended in $PBS^{2+}$. Percentage of fluorescent cells was analyzed by FACSCalibur flow cytometer (Becton Dickinson, CA, USA) with computer assisted evaluation data (CellQuest Pro).

*MHV infection of LR7 cells*

Mouse L cells were seeded at a concentration of $5x10^4$ cells/well and preincubated for 1h with fivefold dilutions of APHS or APHS-d (ranging from 143 to 0.009 μM) in DMEM/10. Afterwards the medium was discarded and the cells were incubated for 1h with MHV-A59 at a multiplicity of infection (M.O.I.) of 0.05 in the presence or absence of APHS or APHS-d in DMEM. Subsequently the cells were washed and incubated with APHS or APHS-d in DMEM/10. After 24h, the cytopatic effect (CPE) of the virus and the cytotoxicity of the compounds were scored by microscopic observation.

*HSV-1 infection of HFF and BHK cells*

This assay was performed as described previously [20]. Briefly, HFF cells were seeded and incubated for 2 days at 37 °C and 5% $CO_2$. Afterwards the medium was removed and the cells were infected with 40-100 PFU/ml of HSV-1 in the presence of increasing concentrations of APHS. Infection was enhanced by centrifugation of the cells for 1h at room temperature. The culture was then incubated for 1 day at 37 °C and 5% $CO_2$.

Subsequently, the medium was aspirated and the HFF monolayer was overlayed with BHK cells and the culture was incubated at 37 °C and 5% $CO_2$. After 24h of incubation, the supernatant was aspirated and the cells were lysed. β-galactosidase activity was detected in the lysates using chlorophenolred-β-D-galactopyranoside (CPRG) (Roche, Almere, The Netherlands).

*Determination of $IC_{50}$ and $TC_{50}$*

The ability of each compound to inhibit HSV-1 replication and cellular viability were expressed as the 50% inhibitory concentration ($IC_{50}$) and the 50% toxic concentration ($TC_{50}$), respectively. $IC_{50}$ and $TC_{50}$ were calculated using the computer software program CalcuSyn for Windows (Biosoft, Cambridge, UK) according to the method of Chou and Talalay [21,22]. This program uses the median-effect equation to produce dose-effect curves:

$$f_a = 1/[1 + (D_m/D)^m] \quad \text{[equation 1]}$$

where $f_a$ represents fraction affected by the dose (reduction of HSV-1 replication or cellular viability at a certain drug concentration expressed in decimals), Dm is the median effect dose (same as $IC_{50}$ or $TC_{50}$), D is the dose of the drug and m is the sigmoidicity coefficient of the dose-effect curve. Data was accepted when the linear correlation coefficient of the median-effect plot based on experimental data was > 0.90.

Results

*Effect of APHS and APHS-d on FIV replication*

Since APHS can inhibit the replication of HIV-1 by interfering with its reverse transcription process, we hypothesize that APHS could also be effective against other retroviruses such as FIV. To assess the effect of APHS and APHS-d on FIV replication, virus replication was assessed by quantifying the levels of the FIV capsid protein p24 in culture supernatants after 6 days of incubation. APHS and APHS-d did not show any effect on FIV replication on these cells (Figure 4.1). APHS and APHS-d concentrations used in this assay were not cytotoxic as determined by the cellular viability assay (data not shown).

Effect of APHS on RSV replication on the HEp-2 cell line

To determine whether APHS could inhibit the replication of the (-) stranded RNA virus RSV, virus replication was quantified by determining the percentage of HEp-2 cells with RSV adherent to their surface. The results are shown in Figure 4.2. RSV infection was measured 4, 5 and 6 days after infection. On day 6 after infection 80% of the cells were RSV-infected. RSV infection was also confirmed by microscopic evaluation of syncytium formation (data not shown). All APHS concentrations tested showed no inhibition of RSV replication on HEp-2 cells. Cellular viability significantly decreased at concentrations above 10 μM as described above. APHS concentrations used in this assay were not cytotoxic as determined by the cellular viability assay (data not shown).

*Effect of APHS and APHS-d on MHV replication*

To assess the effect of APHS and APHS-d on the replication of the (+) stranded RNA virus MHV, virus replication was assessed by CPE scoring after 24h of incubation. CPE were visible in every sample except for the negative control, which indicates a good infection but no effect of APHS or APHS-d (data not shown). All tested concentrations were not cytotoxic as determined by microscopic observation (data not shown).

*Effect of APHS on HSV-1 replication*

Since some antiviral drugs act against both HIV-1 and HSV-1, it was determined whether APHS can also be active against HSV-1. To determine the effect of APHS on the replication of the double-stranded DNA virus HSV-1, an *in vitro* assay was performed as described previously. It was found that APHS is capable of inhibiting the replication of the HSV-1 wild-type strain KOS in a dose-dependent manner. The $IC_{50}$ of APHS in KOS-infected cells was 50 μM (Table 4.1). In addition, APHS is capable of inhibiting the replication of the DP mutant HSV-1 strain $AraA^r8$ in a dose-dependent manner. However, the $IC_{50}$ of APHS in $AraA^r8$-infected cells was 119 μM, which is about two times higher than the $IC_{50}$ for the wild-type (Table 4.1). APHS is not capable of inhibiting the replication of the DP mutant HSV-1 strain $PAA^r5$ or of the TK mutant HSV-1 strain 98.14742-PE/1 (Table 4.1). APHS concentrations used in this assay were not cytotoxic as determined by the cellular viability assay. The $TC_{50}$ of APHS for these cells was found to be 374 μM.

Since APHS can inhibit the replication of HIV-1 by interfering with its reverse transcription process, we hypothesize that APHS could also be active against other retroviruses. However, APHS did not show any effect on FIV replication at the concentrations used. If APHS would target a cellular factor involved in the reverse transcription process it would probably also inhibit the replication of FIV. This result seems to indicate that APHS specifically interferes with the RT of HIV-1 or with other HIV-1 proteins involved in the reverse transcription process. It is known that the NNRTIs currently used in the clinic are only active against HIV-1 and not against HIV-2, SIV, FIV, or other retroviruses apparently because only HIV-1 RT offers the required allowance for interactions of the NNRTIs with their binding pocket, i.e., stacking interactions with the aromatic amino acids (aa) Tyr-181, Tyr-188, Trp-229 and Tyr-318; electrostatic interactions with Lys-101, Lys-103 and Glu-138; van der Waals interactions with Leu-100, Val-106, Tyr-181, Gly-190, Trp-229, Leu-234 and Tyr-318; and hydrogen bonding with the main-chain peptide bonds [8]. In addition, it was previously found that although the aa in the NNRTI-specific pocket of HIV-1 RT display higher similarity to the corresponding FIV RT aa than to HIV-2 RT aa, the wild-type FIV and the FIVp66/HIVp51 chimeric enzyme showed no susceptibility for NNRTI [23]. This suggests that APHS is a NNRTI.

APHS did not inhibit the replication of RSV nor that of MHV. This is not surprising since the mechanism of replication of these viruses is totally different than the mechanism of replication of HIV-1. Interestingly, APHS did inhibit the replication of HSV-1 wild-type strain KOS. This result correlate well with previous studies that showed that HSV can be inhibited by non-nucleoside inhibitors [24,25]. Since RT is also a DP, two HSV-1 strains with mutations in the DP gene, $AraA^r8$ and $PAA^r5$, were also tested. APHS was two times less effective in inhibiting the replication of $AraA^r8$ and could not inhibit the replication of $PAA^r5$ at the tested concentrations. These findings correlate well with the fact that the $IC_{50}$ of ACV for KOS is two and ten times lower than the $IC_{50}$ of ACV for $AraA^r8$ and $PAA^r5$, respectively [26]. This assay strongly suggests that APHS is inhibiting HSV-1 replication by targeting the DP. Although it is known that some antiviral drugs can inhibit both HIV-1 and HSV-1[5], this result came as a surprise because APHS could not inhibit FIV replication at the concentrations tested. It might be that APHS is interacting with regions of the HIV-1 RT, which have a corresponding region in the HSV-1 DP, but not in the FIV RT. Since APHS probably has to be modified in the cell in order to become an active antiviral agent [1] and since TK has the ability to activate certain drugs such as ACV into an active antiherpetic agent, the TK-mutant HSV-1 strain with mutations on the viral TK, 98.14742-PE/1, was also tested. APHS could not inhibit the replication of this HSV-1 strain. Although we do not know how TK modifies APHS, this seems to be a crucial step for APHS conversion into an active HSV-1 antiviral agent. Although this study indicates that APHS is active against HSV-1, more extensive studies are needed before APHS can be considered as a new antiherpetic drug. In particular, more HSV-1 clinical isolates should be tested in order to assess the effect of the natural variation of HSV-1 on the antiviral activity of APHS. In addition, the effect of mutations in the DP or TK gene could be assessed by comparing the antiviral activity of APHS against viruses with the same genetic background but different point mutations in these genes.

Table 4.1.

The effect of APHS on HSV-1 strains KOS, AraA$^r$8, PAA$^r$5 and 98.14742-PE/1 replication in HFF and BHK cells.

| HSV-1 strain | Drug susceptibility | Mutations | $IC_{50}$[a] (μM) |
|---|---|---|---|
| KOS | ACV-sensitive | - | 47 ± 17 |
| AraA$^r$8 | ACV-resistant | DP | 134 ± 15 |
| PAA$^r$5 | PAA- and ACV-resistant | DP | >200 |
| 98.14742-PE/1 | ACV-resistant | TK | >200 |

[a] $IC_{50}$ represents 50% inhibitory concentration. Results are the average of three independent experiments.

References with example 4

1. Pereira,C.F. *et al.* Aspirin-like molecules that inhibit human immunodeficiency virus-1 replication. *Antivir. Res. in press* (2003).

2. De Clercq,E. 2001 ASPET Otto Krayer Award Lecture. Molecular targets for antiviral agents. *J. Pharmacol. Exp. Ther.* 297, 1-10 (2001).

3. Crumpacker,C.S. Mechanism of action of foscarnet against viral polymerases. *Am. J. Med.* 92 , 3S-7S (1992).

4. Tachedjian,G. *et al.* Characterisation of foscarnet-resistant strains of human immunodeficiency virus type 1. *Virology* 212, 58-68 (1995).

5. De Clercq,E. *et al.* A novel selective broad-spectrum anti-DNA virus agent. *Nature* 323, 464-467 (1986).

6. Vahlenkamp,T.W. *et al.* (R)-9-(2-phosphonylmethoxypropyl)-2,6-diaminopurine is a potent inhibitor of feline immunodeficiency virus infection. *Antimicrob.* Agents *Chemother.* 39, 746-749 (1995).

7. Naesens,L., Balzarini,J. & De Clercq,E. The potential of acyclic nucleoside phosphonates as broad-spectrum antiviral agents. *Postepy Biochem.* 41, 347-351 (1995).

8. Jonckheere,H., Anne,J. & De Clercq,E. The HIV-1 reverse transcription (RT) process as target for RT inhibitors. *Med. Res. Rev.* 20, 129-154 (2000).

9. Egberink,H. *et al.* Suppression of feline immunodeficiency virus infection in vivo by 9-(2-phosphonomethoxyethyl)adenine. *Proc. Natl. Acad. Sci. U. S. A* 87, 3087-3091 (1990).

10. Hartmann,K. Feline immunodeficiency virus infection--causative agent of an acquired immunodeficiency syndrome in cats. *Eur. J. Med. Res.* 1, 547-550 (1996).

11. North,T.W., Cronn,R.C., Remington,K.M. & Tandberg,R.T. Direct comparisons of inhibitor sensitivities of reverse transcriptases from feline and human immunodeficiency viruses. *Antimicrob. Agents Chemother.* 34, 1505-1507 (1990).

12. Spaan,W.J., Rottier,P.J., Horzinek,M.C. & van der Zeijst,B.A. Isolation and identification of virus-specific mRNAs in cells infected with mouse hepatitis virus (MHV-A59). *Virology* 108, 424-434 (1981).

13. Gibbs,J.S., Chiou,H.C., Bastow,K.F., Cheng,Y.C. & Coen,D.M. Identification of amino acids in herpes simplex virus DNA polymerase involved in substrate and drug recognition. *Proc. Natl. Acad. Sci. U. S. A* 85, 6672-6676 (1988).

14. Coen,D.M., Fleming,H.E., Jr., Leslie,L.K. & Retondo,M.J. Sensitivity of arabinosyladenine-resistant mutants of herpes simplex virus to other antiviral drugs and mapping of drug hypersensitivity mutations to the DNA polymerase locus. J. *Virol.* 53, 477-488 (1985).

15. Marcy,A.I., Hwang,C.B., Ruffner,K.L. & Coen,D.M. Engineered herpes simplex virus DNA polymerase point mutants: the most highly conserved region shared among alpha-like DNA polymerases is involved in substrate recognition. *J. Virol.* 64, 5883-5890 (1990).

16. Morfin,F., Thouvenot,D., Souillet,G., Michallet,M. & Aymard,M. Aciclovir-resistant (ACV-R) herpes viruses (HSV, VZV) in bone marrow transplantation patients. Abstract. Acta Microbiologica et Immunologica Hungarica 46, 429 (1999).

17. Schaffer,P.A., Aron,G.M., Biswal,N. & Benyesh-Melnick,M. Temperature-sensitive mutants of herpes simplex virus type 1: isolation, complementation and partial characterization. *Virology* 52, 57-71 (1973).

18. Pauwels,R. *et al.* Rapid and automated tetrazolium-based colorimetric assay for the detection of anti-HIV compounds. *J. Virol. Methods* 20, 309-321 (1988).

19. Vahlenkamp,T.W. *et al.* Competitive reverse transcription-polymerase chain reaction for quantitation of feline immunodeficiency virus. *J. Virol. Methods* 52, 335-346 (1995).

20. Stránská,R., Scholl,D.R., Jollick,J.A., Shaw C.J., Polman,M., van Loon,A.M. & Schuurman,R. ELVIRA® HSV-a rapid assay for antiviral resistance testing of herpes simplex virus. Abstract no. 1898. 41st Interscience Conference on Antimicrobial Agents and Chemotherapy, Chicago, Illinois, 16-19 December 2001.

21. Chou,T.C. & Talalay,P. Quantitative analysis of dose-effect relationships: the combined effects of multiple drugs or enzyme inhibitors. *Adv. Enzyme Regul.* 22, 27-55 (1984).

22. Chou,T.C. & Hayball,M.P. CalcuSyn: Windows software for Dose Effect Analysis. (BIOSOFT, Cambridge,UK, 1996).

23. Auwerx,J. *et al.* Chimeric human immunodeficiency virus type 1 and feline immunodeficiency virus reverse transcriptases: role of the subunits in resistance/sensitivity to non-nucleoside reverse transcriptase inhibitors. *Mol. Pharmacol.* 61, 400-406 (2002).

24. Wentland,M.P. *et al.* Antiviral properties of 3-quinolinecarboxamides: a series of novel non-nucleoside antiherpetic agents. *Drug Des Discov.* 15 , 25-38 (1997).

25. Renau,T.E., Wotring,L.L., Drach,J.C. & Townsend,L.B. Synthesis of non-nucleoside analogs of toyocamycin, sangivamycin, and ++thiosangivamycin: influence of various 7-substituents on antiviral activity. *J. Med. Chem.* 39, 873-880 (1996).

26. Stranska,R., van Loon,A.M., Polman,M. & Schuurman,R. Application of real-time PCR for determination of antiviral drug susceptibility of herpes simplex virus. *Antimicrob. Agents Chemother.* 46, 2943-2947 (2002).

## Claims

**1.** A method for determining anti-viral activity of a compound comprising testing of a compound of the general formula

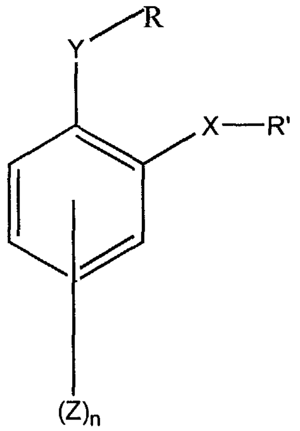

wherein X and Y are independently O, S, SO, $SO_2$, $SO_3$, but preferably O,S, $SO_2$; wherein n is 0, 1, 2, 3, 4, but preferably 1 or 2; wherein R, R' are independently H with the proviso that when R is H, R' is not H, a $C_1$-$C_{10}$, branched or unbranched, substituted or unsubstituted (preferably the substitute is one or more of halogen or $CF_3$), saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, $CH_3$, $CF_3$, $CH_2CL$, $CH_2Br$, $CH_2CH_3$, $(CH_2)_2CH_3$, $(CH_2)_3CH_3$, $(CH_2)_4CH_3$, $(CH_2)_5CH_3$, $(CH_2)_6CH_3$, $CH(CH_3)_2$, $CH(CH_3)_3$. $CH=C=CH_2$, $(CH_2)_2O(CH_2)_3CH_3$, $CH_2HC=CH(CH_2)_3CH_3$, $CH_2C \equiv C(CH_2)_3CH_3$, $CH_2C \equiv C(CH_2)_2CH_3$, $CH_2C \equiv C\text{-}CCH_2CH_3$, $CH_2C \equiv C\text{-}CH_3$ and $CH_2C \equiv CH$ and isomers or homologues thereof and wherein X and/or Y may be linked to R or R' via ether linkages or carbonyl or thiocarbonyl functions such as -(C=O/S)-
and Z is independently R, R', XR, XR', YR or YR'
or a functional equivalent or pharmaceutically acceptable salt or hydrate thereof for its likelihood to modulate a viral polymerase.

**2.** A method according to claim 1 wherein said viral polymerase comprises a reverse transcriptase

**3.** A method according to claim 1 or 2 wherein R' is R.

**4.** A method according to claim 1 to 3 wherein said polymerase is inhibited.

**5.** A method for identifying and obtaining an anti-viral agent comprising testing a first compound in a method according to claim 1, modifying said compound and testing said modified compound for its likelihood to modulate a viral polymerase.

**6.** A method according to claim 5 further comprising selecting a compound capable of inhibiting said polymerase.

**7.** A method according to claim 5 or 6 further comprising testing said modified compound for its capability to act synergistically with another anti-viral agent.

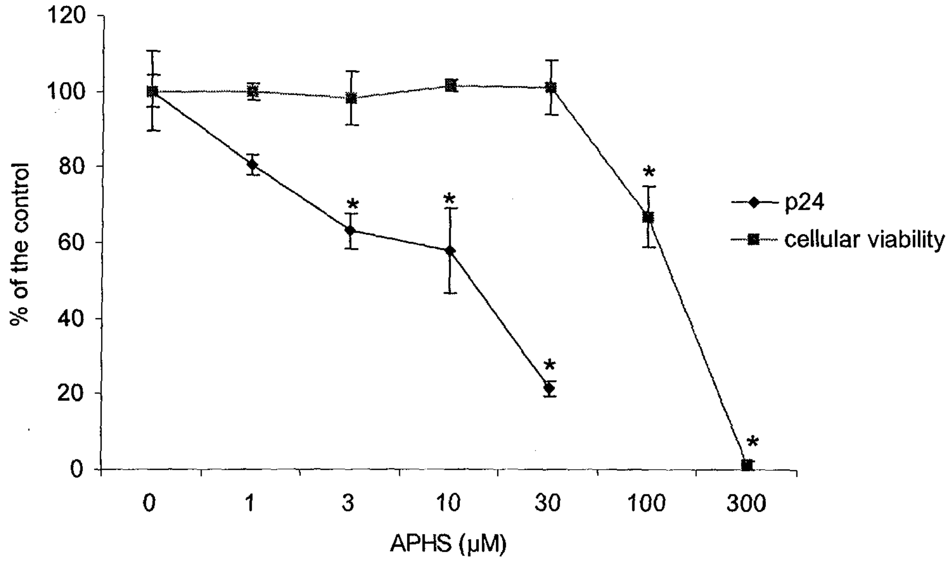
120
100
80
60
40
20
0
% of the control
*
*
*
*
*
p24
cellular viability
0
1
3
10
30
100
300
APHS (µM)

Figure 1.1.

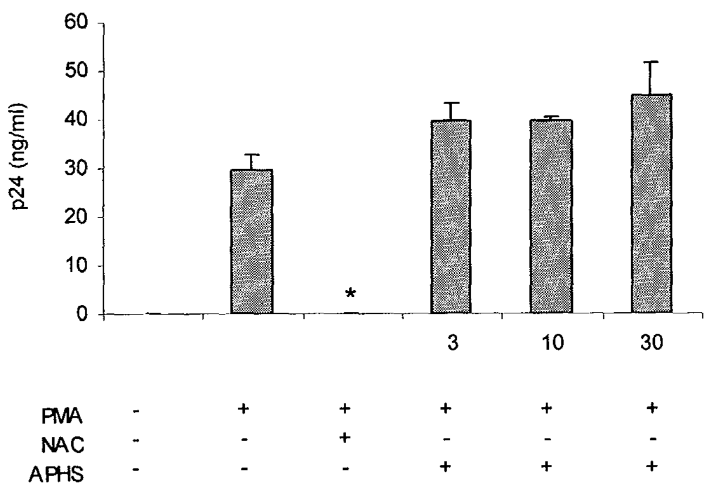


Figure 1.2

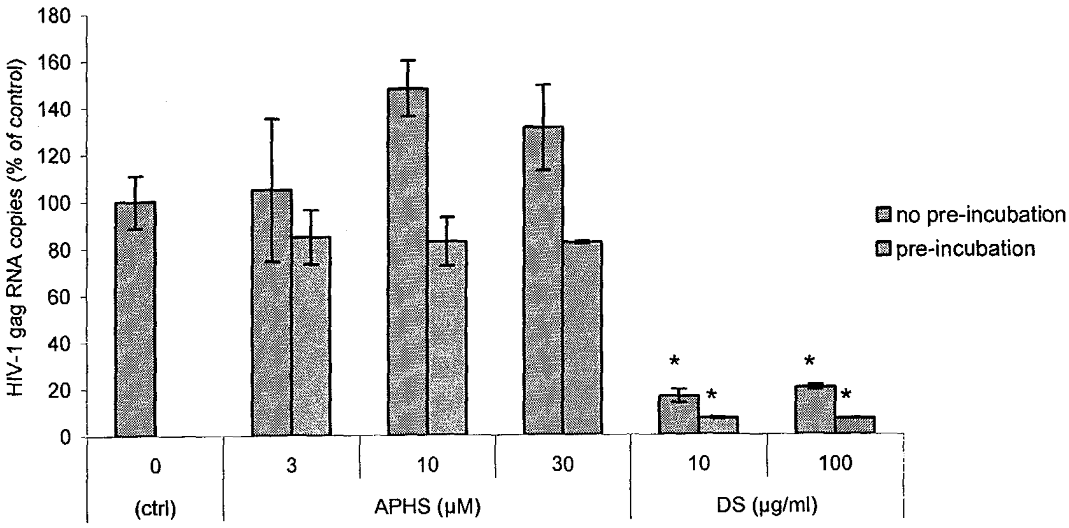
HIV-1 gag RNA copies (% of control)
180
160
140
120
100
80
60
40
20
0
no pre-incubation
pre-incubation
*
*
*
*
0
(ctrl)
3
10
30
APHS (µM)
10
100
DS (µg/ml)

Figure 1.3

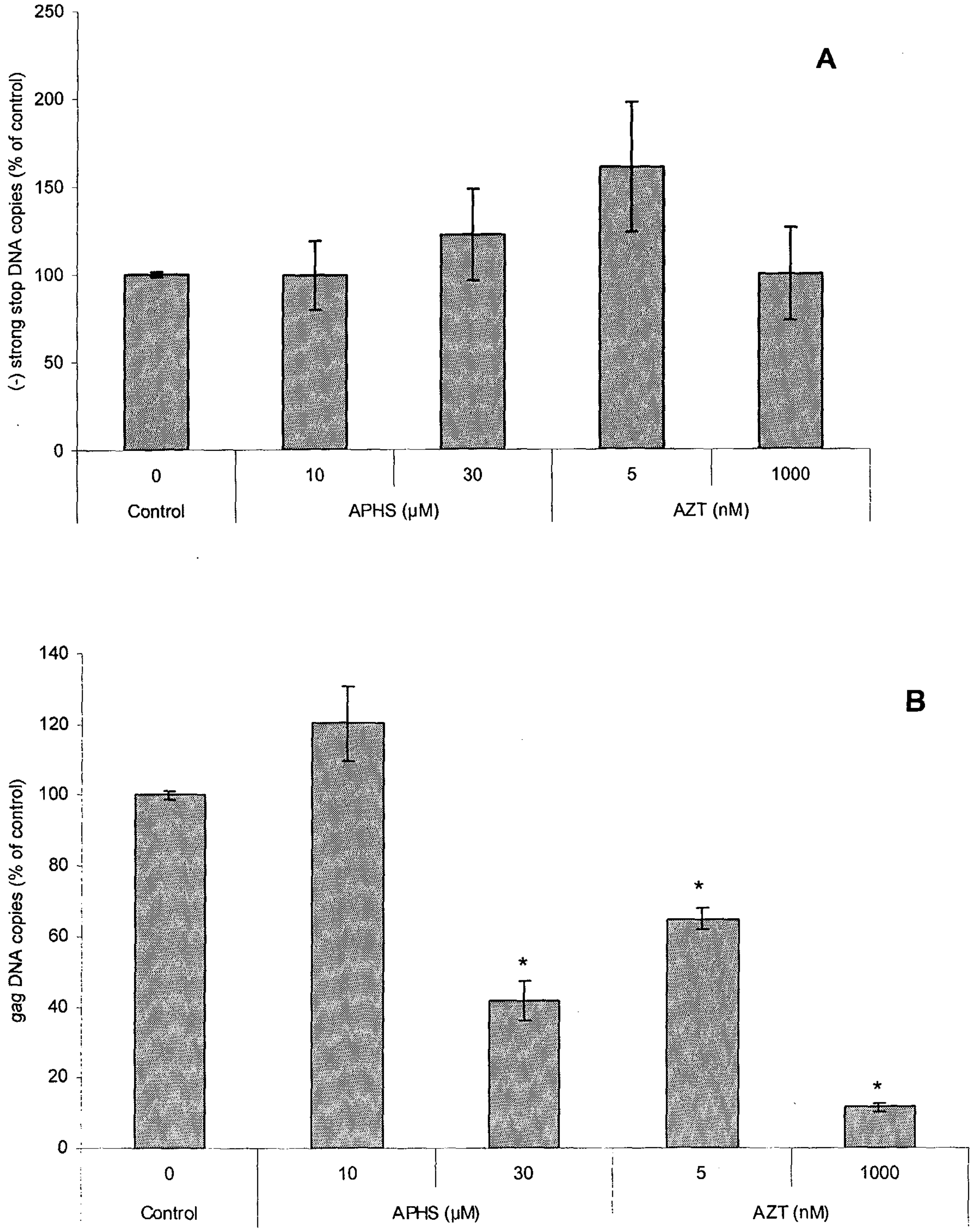
250
200
150
100
50
0
(-) strong stop DNA copies (% of control)
A
0
10
30
5
1000
Control
APHS (µM)
AZT (nM)
140
120
100
80
60
40
20
0
gag DNA copies (% of control)
B
*
*
*
0
10
30
5
1000
Control
APHS (µM)
AZT (nM)

Figure 1.4

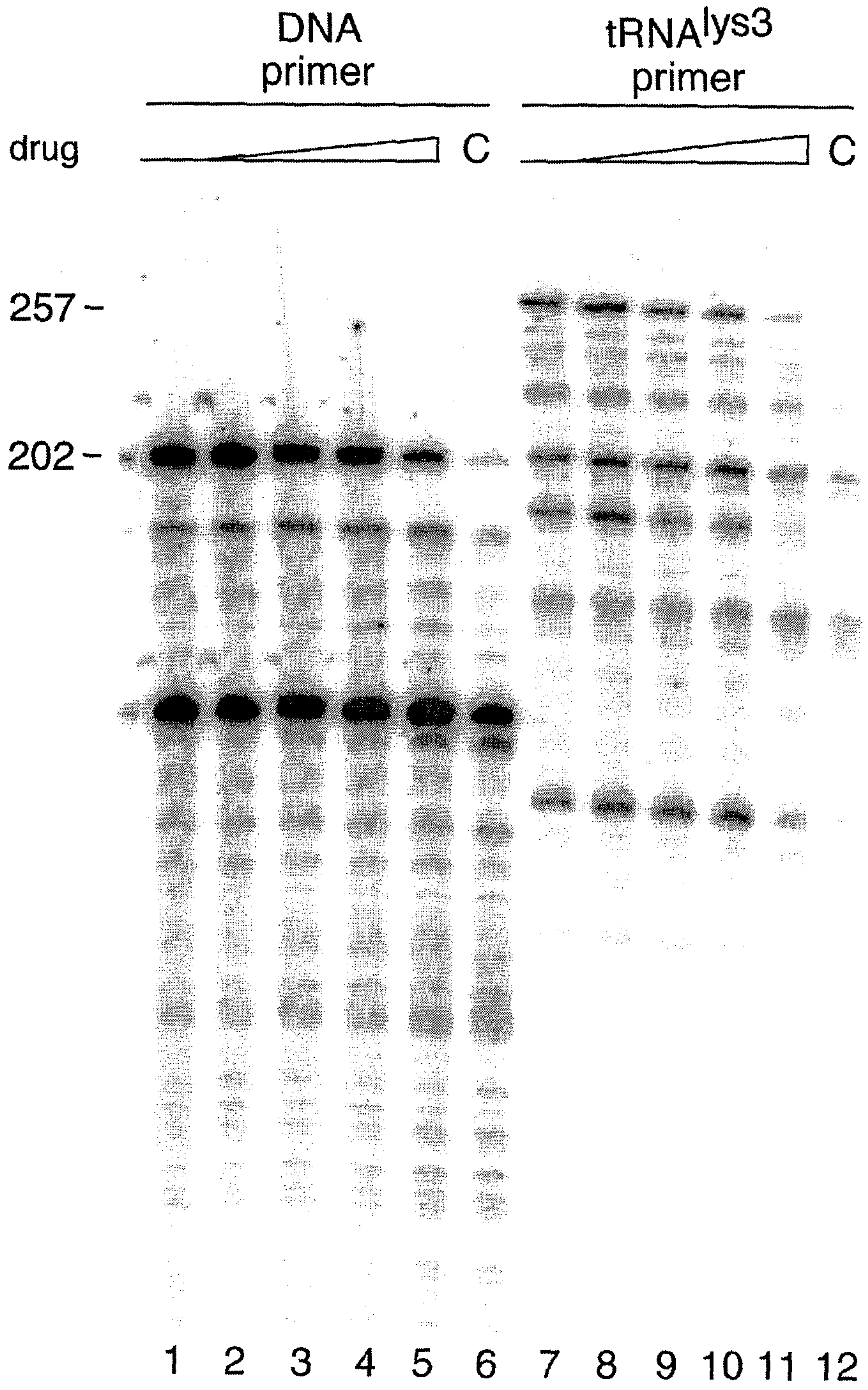
DNA
primer
tRNA$^{lys3}$
primer
drug
C
C
257
202
1 2 3 4 5 6 7 8 9 10 11 12

Figure 1.5

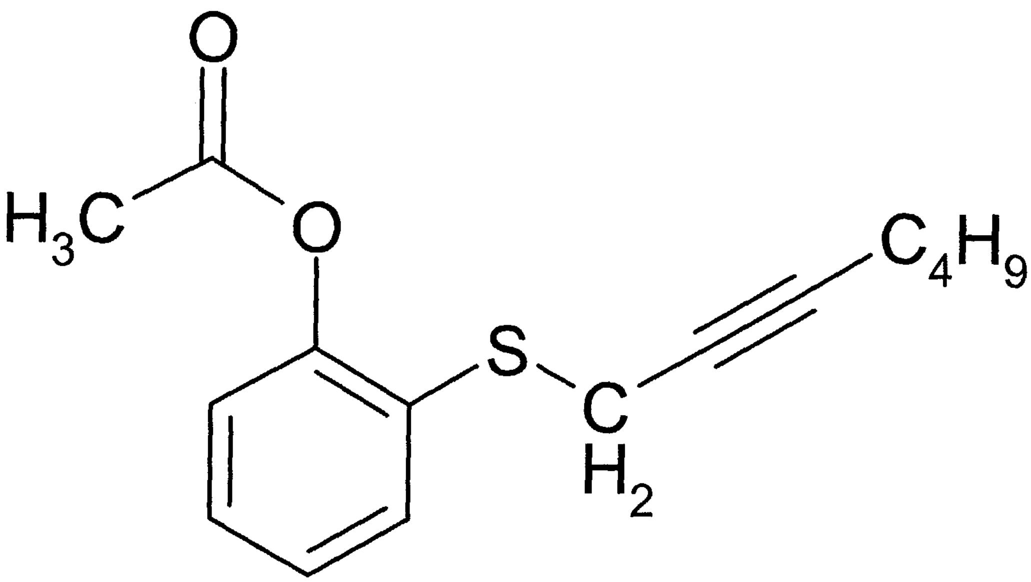

Figure 3.1

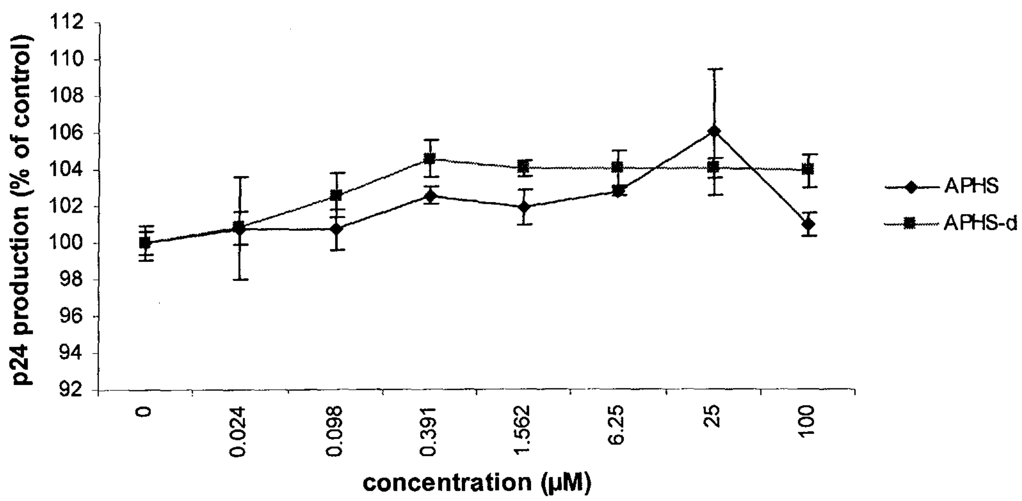
p24 production (% of control)
112
110
108
106
104
102
100
98
96
94
92
0
0.024
0.098
0.391
1.562
6.25
25
100
concentration (µM)
APHS
APHS-d

Figure 4.1

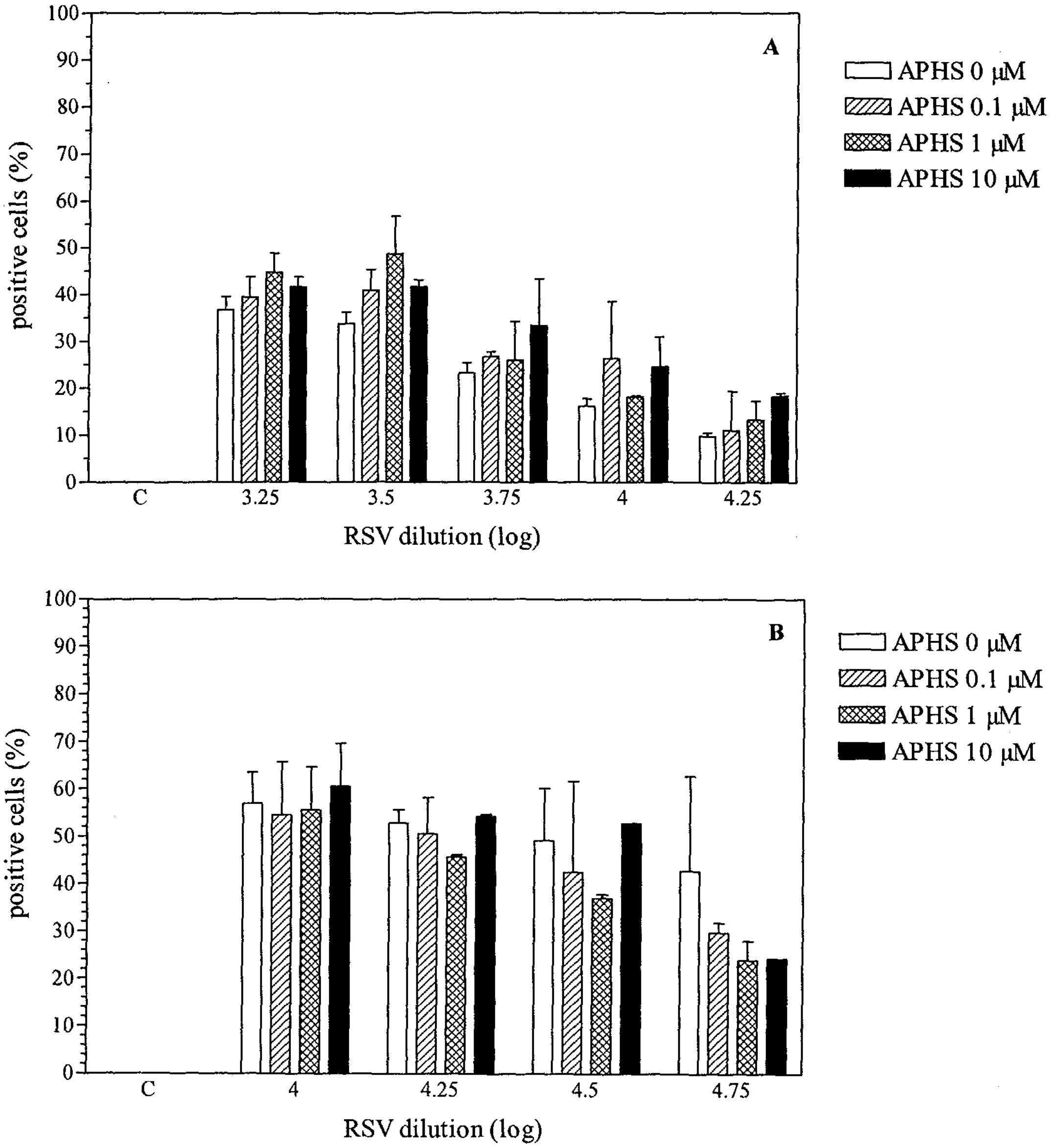
A
APHS 0 μM
APHS 0.1 μM
APHS 1 μM
APHS 10 μM
positive cells (%)
0
10
20
30
40
50
60
70
80
90
100
C
3.25
3.5
3.75
4
4.25
RSV dilution (log)
B
APHS 0 μM
APHS 0.1 μM
APHS 1 μM
APHS 10 μM
positive cells (%)
C
4
4.25
4.5
4.75
RSV dilution (log)

Figure 4.2

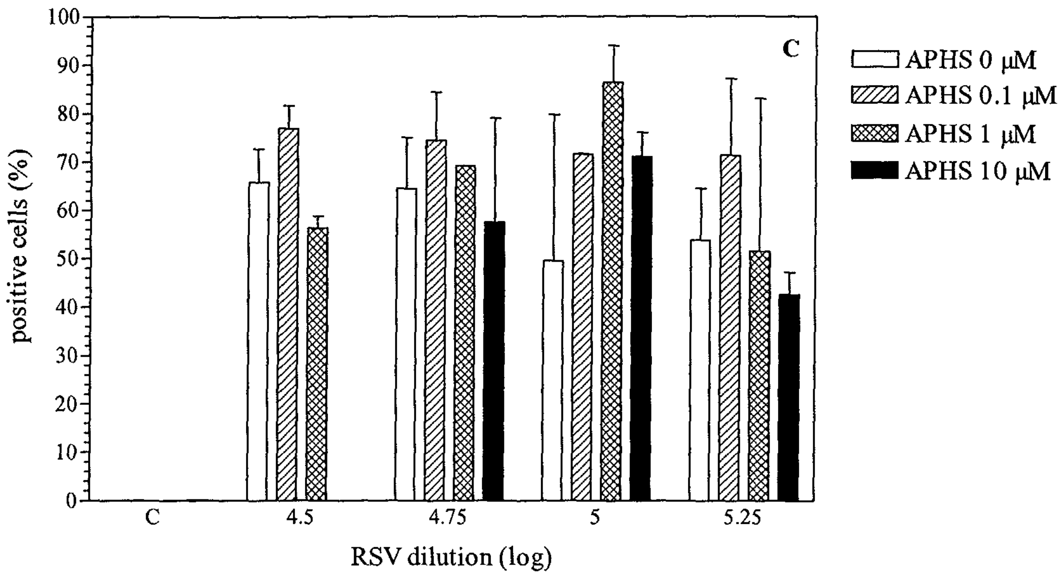
100
90
80
70
60
50
40
30
20
10
0
positive cells (%)
C
C
4.5
4.75
5
5.25
RSV dilution (log)
APHS 0 μM
APHS 0.1 μM
APHS 1 μM
APHS 10 μM

Figure 4.2

European Patent Office

# PARTIAL EUROPEAN SEARCH REPORT

which under Rule 45 of the European Patent Convention shall be considered, for the purposes of subsequent proceedings, as the European search report

Application Number EP 03 07 5596

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 01 68086 A (UNIV UTRECHT ;NOTTET JOHANNES SERVATIUS LEON (NL); UNI MEDISCH CT) 20 September 2001 (2001-09-20)<br>* page 6, line 26 - line 32 *<br>* the whole document *<br>--- | 1-7 | C12Q1/25 |
| D,T | PEREIRA C F ET AL: "Aspirin-like molecules that inhibit human immunodeficiency virus 1 replication" ANTIVIRAL RESEARCH 01 MAY 2003 NETHERLANDS,<br>vol. 58, no. 3, 1 May 2003 (2003-05-01), pages 253-263, XP001159799<br>ISSN: 0166-3542<br>* the whole document *<br>----- | 1-7 | |

TECHNICAL FIELDS SEARCHED (Int.Cl.7)

C12Q
G01N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 21 July 2003 | Tuynman, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

## INCOMPLETE SEARCH SHEET C

Application Number
EP 03 07 5596

Claim(s) searched incompletely:
1-7

Reason for the limitation of the search:

Present claims 1-7 relate to an extremely large number of possible compounds. In fact, the claims contain so many possible permutations that a lack of clarity (and conciseness) within the meaning of Article 84 EPC arises to such an extent as to render a meaningful search of the claims impossible. Consequently, the search has been carried out for those parts of the application which do appear to be clear (and concise), namely those compounds mentioned in Table 1.2 of the description and the o-(pentylthio)phenol mentioned on page 8 lines 17-20.

## ANNEX TO THE EUROPEAN SEARCH REPORT ON EUROPEAN PATENT APPLICATION NO. EP 03 07 5596

This annex lists the patent family membersrelating to the patent documents cited in the above-mentioned European search report.
The members are as contained in the European Patent Office EDP file on
The European Patent Office is in no way liable for these particulars which are merely given for the purpose of information.

21-07-2003

| Patent document cited in search report | Publication date | Patent family member(s) | Publication date |
|---|---|---|---|
| WO 0168086 A | 20-09-2001 | AU 4485101 A | 24-09-2001 |
| | | AU 5856100 A | 22-01-2001 |
| | | CA 2373879 A1 | 11-01-2001 |
| | | CA 2403358 A1 | 20-09-2001 |
| | | EP 1194141 A1 | 10-04-2002 |
| | | EP 1263429 A1 | 11-12-2002 |
| | | JP 2003503455 T | 28-01-2003 |
| | | WO 0101985 A1 | 11-01-2001 |
| | | WO 0168086 A1 | 20-09-2001 |

EPO FORM P0459

For more details about this annex : see Official Journal of the European Patent Office, No. 12/82